# FASCICULE DE BREVET EUROPEEN

(11) **EP 1 758 621 B1**
(45) Date de publication et mention de la délivrance du brevet: **11.08.2010**
(21) Numéro de dépôt: 05778669.1
(22) Date de dépôt: 21.06.2005
(51) Int. Cl.: A61L 27/34, A61L 27/54

(54) **BIOMATERIAUX BIOACTIFS POUR LE RELARGAGE CONTROLE DE PRINCIPES ACTIFS**
BIOAKTIVE BIOMATERIALIEN ZUR KONTROLLIERTEN WIRKSTOFFFREISETZUNG
BIOACTIVE BIOMATERIALS FOR CONTROLLED DELIVERY OF ACTIVE PRINCIPLES

(30) Priorité: 21.06.2004 FR 0406708
(43) Date de publication de la demande: 07.03.2007
(73) Titulaire: Centre National de la Recherche Scientifique, 75794 Paris Cedex 16 (FR); INSERM (Institut National de la Santé et de la Recherche Medicale), 75654 Paris Cedex 13 (FR); UNIVERSITE DE BORDEAUX I, 33405 Talence Cedex (FR); Ecole Nationale Superieure de Chimie Et de Physique de Bordeaux 1, 33607 Pessac Cedex (FR)
(72) Inventeur: DURRIEU, Marie-Christine, F-33140 Villenave d'Ornon (FR); QUEMENER, Damien, F-34000 Montpellier (FR); BAQUEY, Charles, Vincent, F-33185 Le Haillan (FR); SABAUT-HEROGUEZ, Valérie, F-33700 Merignac (FR)
(74) Mandataire: Colombet, Alain André
(86) Numéro de dépôt international: PCT/FR2005/001545
(87) Numéro de publication internationale: WO 2006/008386

(56) Documents cités:
- EP-A- 1 371 699
- US-A- 5 782 908
- CHEMTOB ABRAHAM ET AL: "Comparative behavior of polybutadiene and polynorbornene-based latices prepared by dispersion ring-opening metathesis polymerization with a poly(ethylene oxide) macromonomer" J POLYM SCI PART A; JOURNAL OF POLYMER SCIENCE, PART A: POLYMER CHEMISTRY JUN 1 2004, vol. 42, no. 11, 1 juin 2004 (2004-06-01), pages 2705-2716, XP002318811
- CHEMTOB ABRAHAM ET AL: "Dispersion ring-opening metathesis polymerization of norbornene using PEO-based stabilizers" MACROMOLECULES; MACROMOLECULES DEC 3 2002, vol. 35, no. 25, 3 décembre 2002 (2002-12-03), pages 9262-9269, XP002318812

## Description

La présente invention a pour objet des biomatériaux bioactifs pour le relargage contrôlé de principes actifs, ainsi que leur procédé de synthèse, et leurs utilisations.

L'objectif général de la présente invention est de doter les dispositifs implantables de la capacité de s'opposer au développement de différents processus, infectieux et/ou inflammatoires qui peuvent suivre leur mise en place.

Aujourd'hui, pour palier ces effets sont proposés l'administration d'un médicament par voie générale (**A**) ainsi que l'administration locale d'antibiotiques en chirurgie osseuse (**B**).

**A** - Lorsqu'un médicament est administré par voie générale, il se distribue dans tout l'organisme et sa concentration sur le site visé (c'est-à-dire susceptible d'être le siège d'un processus infectieux et / ou inflammatoire et / ou néoplasique) ne peut dépasser le seuil d'efficacité que si la dose administrée est suffisamment élevée au risque d'exposer le patient à des effets toxiques.

Les substances pharmacologiquement administrées par voie orale ou parentérale présentent souvent une demi-vie trop courte et des risques de toxicité générale pour atteindre l'effet local recherché.

**B**- Depuis 1970, les ciments aux antibiotiques sont utilisés dans la chirurgie prothétique articulaire. En France, il existe 2 préparations commercialisées utilisant soit la gentamycine, soit une association d'érythromycine et de colimycine. On peut également préparer du « ciment aux antibiotiques » notamment à la vancomycine en salle d'opération dans des conditions non standardisées. Le facteur limitant de cette méthode est la libération non contrôlée (en terme de concentration et de durée) du principe actif utilisé. En effet, la cinétique de libération du principe actif n'est pas maîtrisée puisque aucun dispositif ne permet d'ajuster son relargage et donc de pérenniser son action sur une durée prédéfinie. De plus, une partie du principe actif n'est pas libérée puisqu'il se trouve piégé trop profondément dans le ciment.

Pour pallier ces inconvénients, se développent des systèmes de délivrance de principes actifs, appelés "Drug delivery system (DDS)". Le principe de ces DDS est de délivrer des substances pharmacologiquement actives in situ, de façon prolongée, régulière, en quantité suffisante et non toxique.

Par ailleurs, des polymères stimulables, c'est-à-dire sensibles à un stimuli extérieur tel qu'une variation de pH ou de température, présentant des fonctions réactives obtenues par encapsulation ou adsorption des principes actifs directement dans le matériau ou dans des billes elles mêmes adsorbées ou greffées sur le matériau, ont déjà été décrits.

Toutefois, l'adsorption ne permet pas une libération contrôlée du principe actif. Quant à l'encapsulation, si elle peut permettre une libération contrôlée du principe actif, elle est en revanche incompatible avec une utilisation prolongée et/ou lorsque le matériau est soumis à de fortes contraintes (flux, frottement...).

Des nanoparticules polymères réactives obtenues par greffage covalent de principes actifs sur la nanoparticule fonctionnalisée ont également déjà été décrites. Cependant, la synthèse de telles nanoparticules se fait en deux étapes (synthèse du latex puis réaction avec le principe actif) et donc sans contrôle direct du greffage (nombre de fonctions introduites aléatoire). Par ailleurs ces nanoparticules ne possèdent pas conjointement le principe actif et des sites d'ancrages permettant une libération en un endroit spécifique du principe actif. Ces matériaux sont le plus souvent destinés à la vectorisation ou aux tests immunologiques.

Le but de la présente invention est de proposer des biomatériaux permettant la libération contrôlée, au niveau du site d'implantation de ces biomatériaux (sur une période de temps ajustable), d'un principe actif fixé de manière covalente en surface de ce dernier grâce à l'ancrage chimique de particules sphériques (notamment de nanoparticules) fonctionnalisées par le principe actif. Une réaction de clivage de la liaison particule-principe actif, actionnée par le contact du matériau avec le milieu physiologique ou par une modification du pH, libère de façon contrôlée la molécule bioactive sous sa forme native. Le concept peut être étendu à la délivrance locale de facteurs aptes à réguler la relation entre un implant et les tissus qui l'entourent. Le principal champ d'application est le domaine biomédical et plus précisément les biomatériaux utilisés en chirurgie vasculaire, endovasculaire (stent) et osseuse.

Ainsi, la présente invention résulte de la mise en évidence du fait qu'il est possible de fixer à la surface de biomatériaux des particules polymériques sphériques bioactives et stimulables, c'est-à-dire sensibles à un stimuli extérieur tel qu'une variation de pH ou de température, présentant à leur périphérie des fonctions réactives de type acide, amine, alcool, chlorure d'acide, ces particules étant obtenus en une seule étape. Avantageusement, la liaison du principe actif sur le matériau est une liaison hydrolysable sous l'effet du pH et/ou de la température.

L'invention concerne des biomatériaux comprenant un matériau support à la surface duquel sont liées de façon covalente des particules sphériques d'un diamètre compris entre 10nm et 100µm, lesdites particules étant constituées par des chaînes polymère contenant environ 30 à 10000 unités monomères, identiques ou différentes, dérivées de la polymérisation d'alcènes monocycliques dont le nombre d'atomes de carbone constitutifs du cycle est d'environ 4 à 12, ou d'alcènes polycycliques dont le nombre total d'atomes de carbone constitutifs des cycles est d'environ 6 à 20, lesdites unités monomères étant telles que :
- au moins environ 0,5 % d'entre elles sont substituées par une chaîne R comprenant un polyoxyde d'éthylène de formule (A) le cas échéant lié de façon covalente auxdites unités monomères via un pont hydrolysable

   -(CH₂-CH₂-O)ₙ-X (A)

   dans laquelle n représente un nombre entier d'environ 50 à 340, notamment de 70 à 200, et X représente une chaîne alkyle ou alkyloxy d'environ 1 à 10 atomes de carbone, comprenant une fonction réactive du type OH, halogène, NH₂, C(O)X₁ dans laquelle X₁ représente un atome d'hydrogène, d'halogène, un groupe OR' ou NHR' dans lequel R' représente un atome d'hydrogène ou une chaîne hydrocarbonée d'environ 1 à 10 atomes de carbone, substituée ou non, ladite fonction réactive étant susceptible de se lier à une fonction réactive située sur le ledit matériau support afin d'assurer la liaison covalente entre ledit matériau et lesdites particules,
- et au moins environ 0,5 % d'entre elles sont substituées par une chaîne R comprenant un polyoxyde d'éthylène de formule (A) susmentionnée dans laquelle ladite fonction réactive est engagée dans une liaison avec un principe actif, ou une molécule biologique telle qu'une protéine, lesdites chaînes R étant liées de façon covalente auxdits monomères.

L'invention a plus particulièrement pour objet des biomatériaux tels que définis ci-dessus, caractérisés en ce que les unités monomères sont dérivées de la polymérisation d'alcènes monocycliques, et sont de formule (Z1) suivante

=[CH-R₁-CH]= (Z1)

dans laquelle R₁ représente une chaîne hydrocarbonée de 2 à 10 atomes de carbone, saturée ou non, lesdites unités monomères étant le cas échéant substituées par une chaîne R, ou directement par un groupe X, tels que définis ci-dessus.

L'invention concerne plus particulièrement des biomatériaux tels que définis ci-dessus, caractérisés en ce que les alcènes monocycliques dont sont issues les unités monomères sont :
- le cyclobutène conduisant à un polymère comprenant des unités monomères de formule (Z1a) suivante : 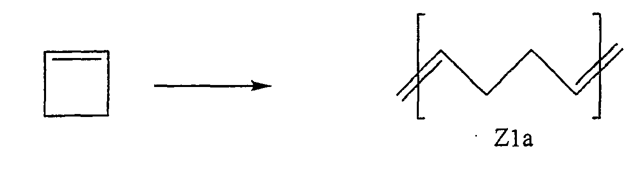
- le cyclopentène conduisant à un polymère comprenant des unités monomères de formule (Z1b) suivante : 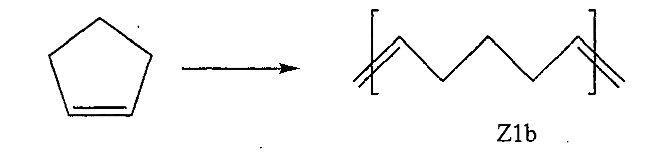
- le cyclopentadiène conduisant à un polymère comprenant des unités monomères de formule (Z1c) suivante 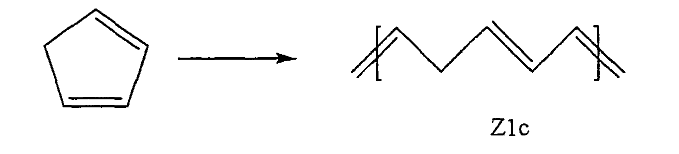
- le cyclohexène conduisant à un polymère comprenant des unités monomères de formule (Z1d) suivante 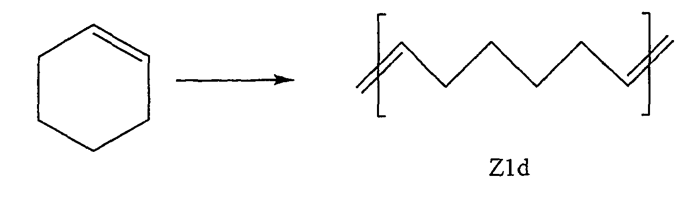
- le cyclohexadiène conduisant à un polymère comprenant des unités monomères de formule (Z1e) suivante 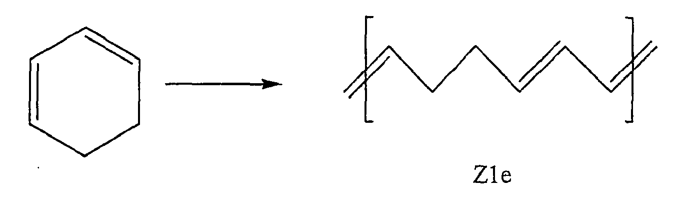
- le cycloheptène conduisant à un polymère comprenant des unités monomères de formule (Z1f) suivante 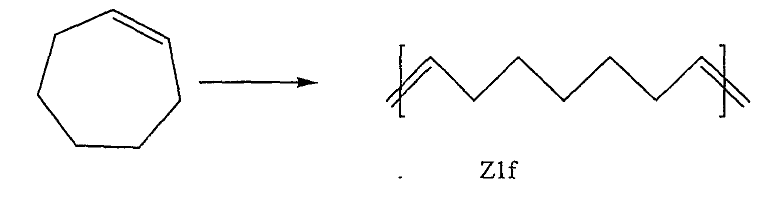
- le cyclooctène conduisant à un polymère comprenant des unités monomères de formule (Z1h) suivante 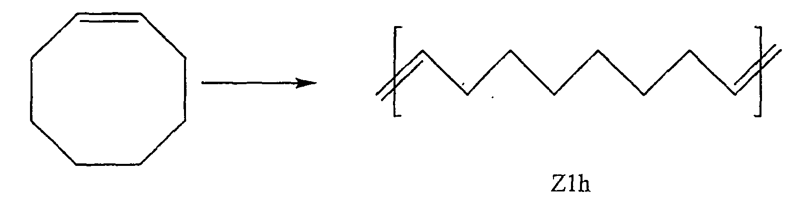
- le cyclooctapolyène, notamment le cycloocta-1,5-diene conduisant à un polymère comprenant des unités monomères de formule (Zli) suivante 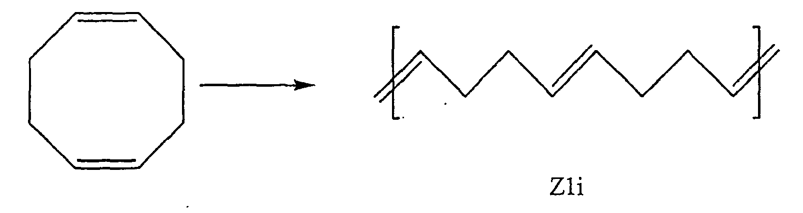
- le cyclononène conduisant à un polymère comprenant des unités monomères de formule (Z1j) suivante 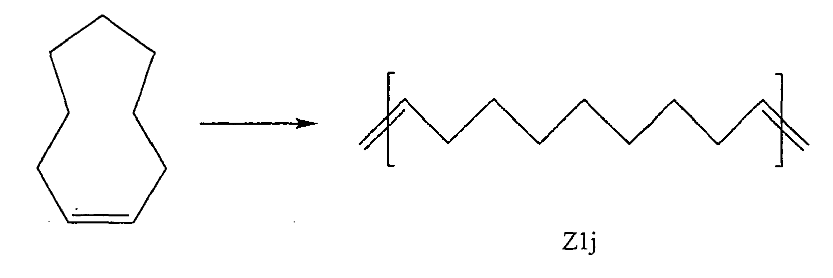
- le cyclononadiène conduisant à un polymère comprenant des unités monomères de formule (Z1k) suivante 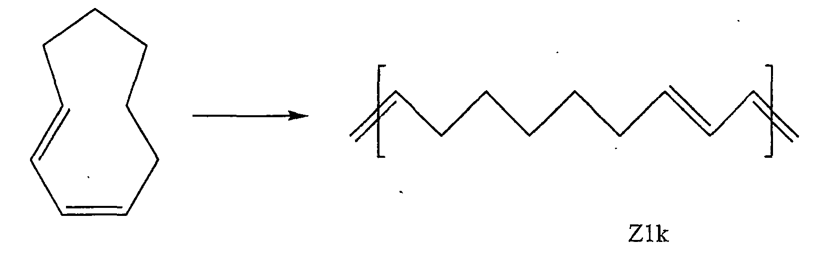
- le cyclodécène conduisant à un polymère comprenant des unités monomères de formule (Z11) suivante 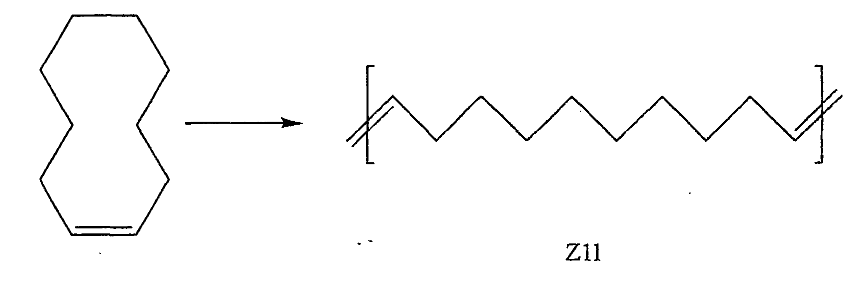
- le cyclodéca-1,5-diène conduisant à un polymère comprenant des unités monomères de formule (Z1m) suivante 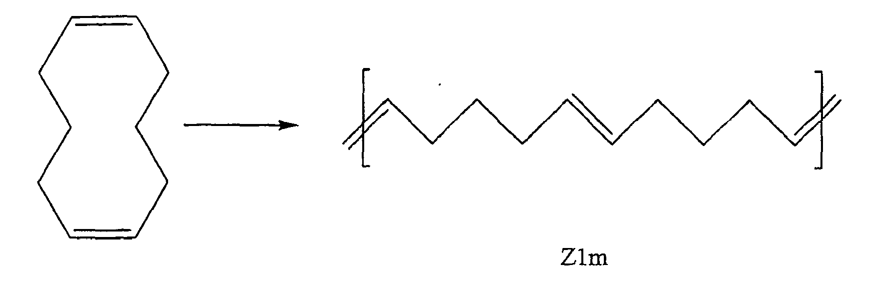
- le cyclododécène conduisant à un polymère comprenant des unités monomères de formule (Z1n) suivante 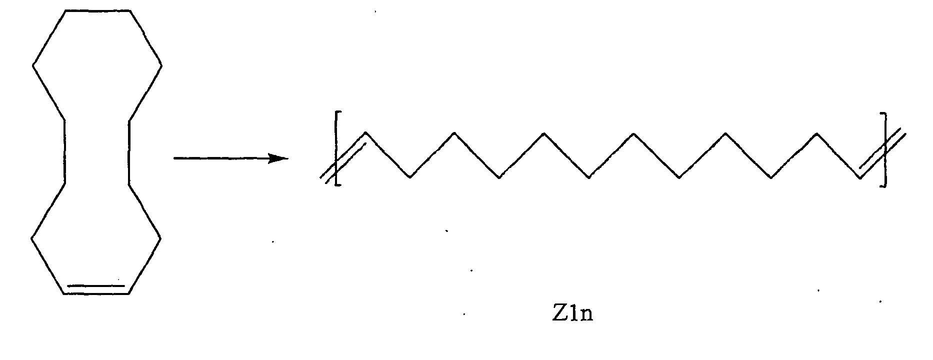
- ou encore l'acétate du 2,3,4,5-tétrahydrooxepin-2-yl, le cyclopentadécène, le paracyclophane, le ferrocenophane.

L'invention concerne également des biomatériaux tels que définis ci-dessus, caractérisés en ce que les unités monomères sont dérivées de la polymérisation d'alcènes polycycliques, et sont :
- de formule (Z2) suivante

   =[CH-R₂-CH]= (Z2)

   dans laquelle R₂ représente :
   * un cycle de formule 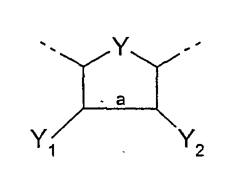 dans laquelle :
      . Y représente -CH₂-, ou un hétéroatome, ou un groupe -CHR-, ou un groupe -CHX-, R et X étant tels que définis ci-dessus,
      . Y1 et Y2, indépendamment l'un de l'autre, représentent H, ou une chaîne R, ou un groupe X susmentionnés, ou forment en association avec les atomes de carbone qui les portent un cycle de 4 à 8 atomes de carbone, ce cycle étant le cas échéant substitué par une chaîne R, ou un groupe X susmentionnés,
      . a représente une simple ou double liaison,
   * ou un cycle de formule 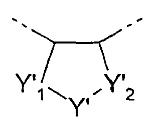 dans laquelle :
      . Y' représente -CH₂-, ou un hétéroatome, ou un groupe -CHR-, ou un groupe -CHX-, R et X étant tels que définis ci-dessus,
      . Y'1 et Y'2, indépendamment l'un de l'autre, représentent -CH₂-, ou un groupe -C(O), ou un groupe -COR, ou un groupe -C-OX, R et X étant tels que définis ci-dessus,
- de formule (Z3) suivante 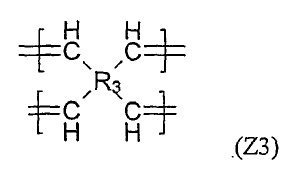 dans laquelle R₃ représente :
   * un cycle de formule 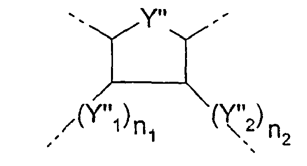 dans laquelle :
      . n1 et n2, indépendamment l'un de l'autre, représentent 0 ou 1,
      . Y" représente -CH₂-, ou un groupe -CHR-, ou un groupe -CHX-, R et X étant tels que définis ci-dessus,
      . Y"1 et Y"2, indépendamment l'un de l'autre, représentent une chaîne hydrocarbonée de 0 à 10 atomes de carbone,
   * ou un cycle de formule 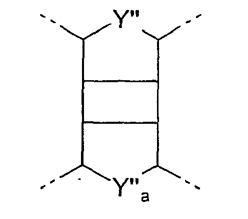 dans laquelle Y" et Y"a, indépendamment l'un de l'autre, représentent -CH₂-, ou un groupe -CHR-, ou un groupe -CHX-, R et X étant tels que définis ci-dessus.
      • ou un cycle de formule 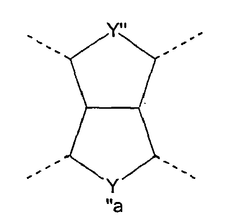
dans laquelle Y" et Y"a, indépendamment l'un de l'autre, représentent -CH₂-, ou un groupe -CHR-, ou un groupe -CHX-, R et X étant tels que définis ci-dessus.

L'invention a plus particulièrement pour objet des biomatériaux tels que définis ci-dessus, caractérisés en ce que les alcènes polycycliques dont sont issues les unités monomères sont :
- les monomères contenant un cycle cyclobutène conduisant à un polymère comprenant des unités monomères de formule (Z2a) suivante : 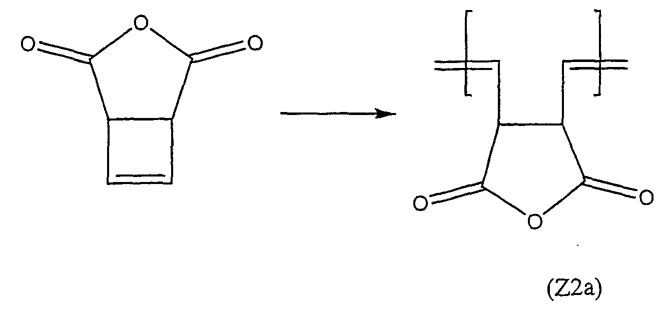
- les monomères contenant un cycle cyclopentène conduisant à un polymère comprenant des unités monomères de formule (Z2b) suivante : 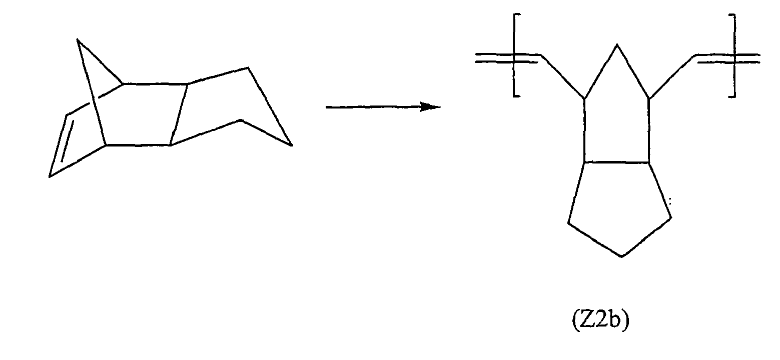
- le norbornène (bicyclo[2.2.1]hept-2-ène) conduisant à un polymère comprenant des unités monomères de formule (Z2c) suivante : 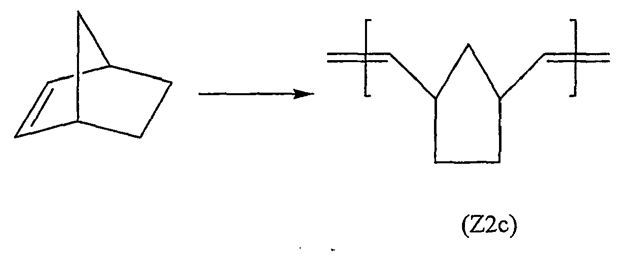
- le norbornadiène conduisant à un polymère comprenant des unités monomères de formule (Z2d) suivante : 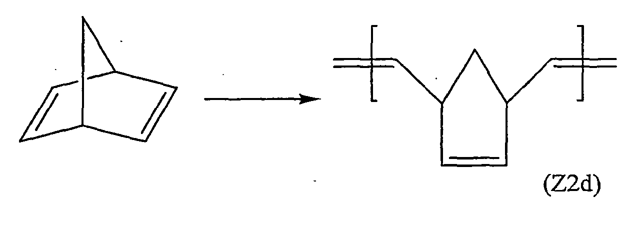
- le 7-oxanorbornène conduisant à un polymère comprenant des unités monomères de formule (Z2e) suivante : 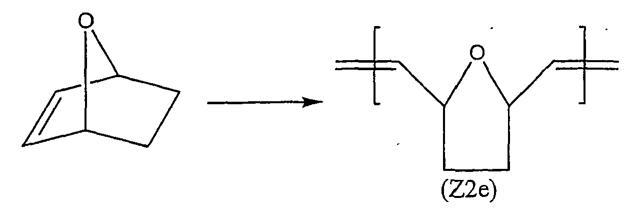
- le 7-oxanorbornadiène conduisant à un polymère comprenant des unités monomères de formule (Z2f) suivante : 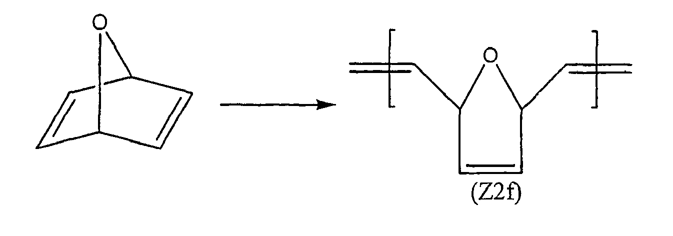
- le dimère du norbornadiène conduisant à un polymère comprenant des unités monomères de formule (Z3a) suivante : 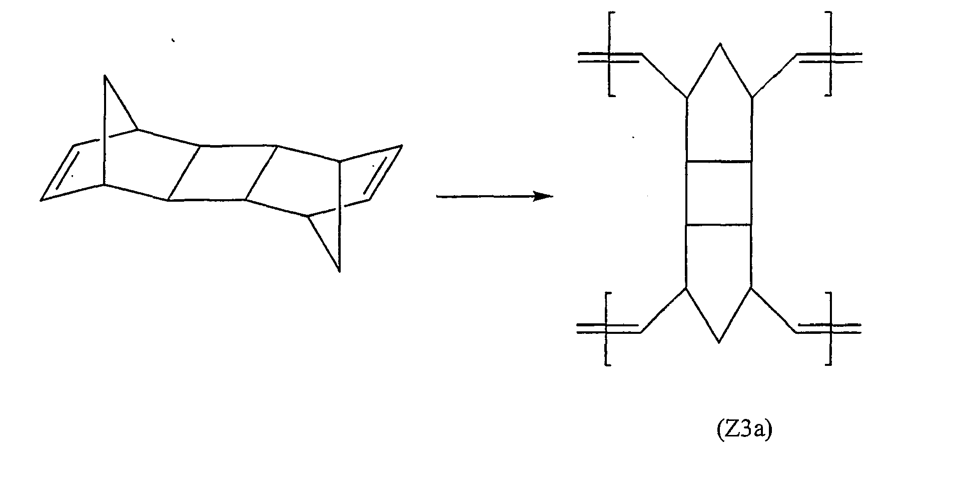
- le dicyclopentadiène conduisant à un polymère comprenant des unités monomères de formule (Z3b) suivante : 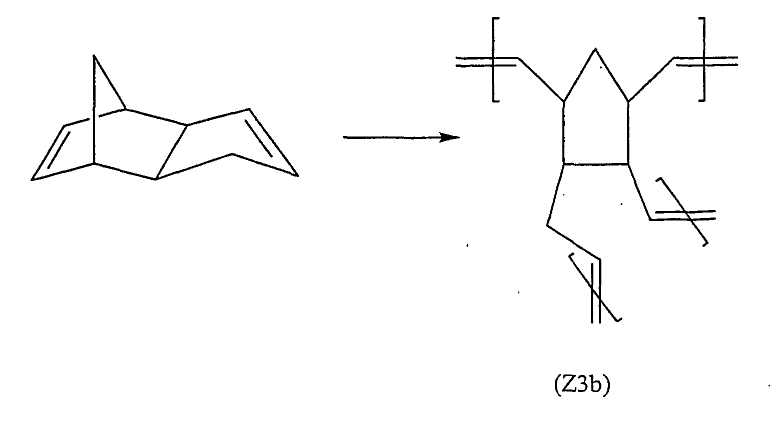
- le tetracyclododécadiene conduisant à un polymère comprenant des unités monomères de formule (Z3c) suivante : 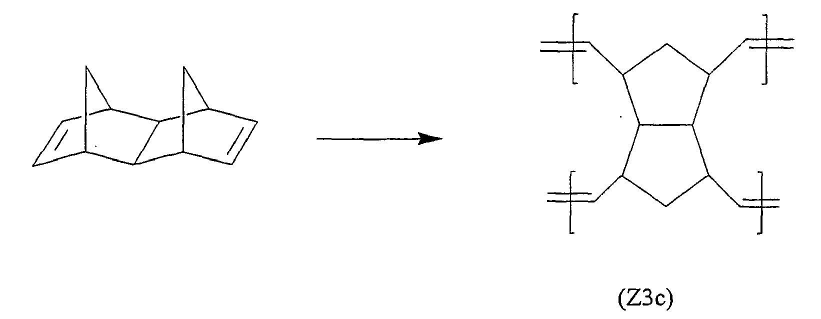
- ou le bicyclo[5.1.0]oct-2-ène, le bicyclo[6.1.0]non-4-ène.

L'invention concerne plus particulièrement des biomatériaux préférés tels que définis ci-dessus, caractérisés en ce que les alcènes mono ou polycycliques dont sont issues les unités monomères sont :
- le norbornène (bicyclo[2.2.1]hept-2-ène) conduisant à un polymère comprenant des unités monomères de formule (Z2c),
- le tetracyclododécadiene conduisant à un polymère comprenant des unités monomères de formule (Z3c),
- le dicyclopentadiène conduisant à un polymère comprenant des unités monomères de formule (Z3b),
- le dimère du norbornadiène conduisant à un polymère comprenant des unités monomères de formule (Z3a),
- le cycloocta-1,5-diene conduisant à un polymère comprenant des unités monomères de formule (Z1i).

L'invention a plus particulièrement pour objet des biomatériaux tels que définis ci-dessus, caractérisés en ce qu'ils comprennent :
- entre environ 0,5 % jusqu'à 100 % d'unités monomères substituées par une chaîne R telle que définie ci-dessus, ladite chaîne R étant identique pour ces monomères, et comprenant une fonction réactive susceptible de se lier à une fonction réactive située sur le ledit matériau support afin d'assurer la liaison covalente entre ledit matériau et lesdites particules,
- et entre environ 0,5 % et 99,5 % d'unités monomères substituées par une chaîne R telle que définie ci-dessus, ladite chaîne R étant identique pour ces monomères, dans laquelle ladite fonction réactive est engagée dans une liaison avec un principe actif, ou une molécule biologique telle qu'une protéine,

- et/ou entre environ 0,5 % et 99,5 % d'unités monomères substituées directement par un groupe X tel que défini ci-dessus, ce groupe X de ces monomères étant identique ou différent du groupe X de la chaîne R des monomères précédents,
- et/ou entre environ 1 % et 99,5 % d'unités monomères non substituées,
le total des pourcentages des différents monomères susmentionnés faisant 100 %.

L'invention concerne plus particulièrement des biomatériaux tels que définis ci-dessus, caractérisés en ce que la ou les chaînes R substituant les monomères sont représentées par la formule

-CH₂-O-(CH₂-CH₂-O)ₙ-CH₂-CH₂-O-X

dans laquelle n est tel que défini ci-dessus, et X représente H, -CH₂-COOH, -CH₂-COCl, -CH₂-COY, Y représentant un principe actif, ou une molécule biologique telle qu'une protéine.

L'invention concerne également des biomatériaux tels que définis ci-dessus, caractérisés en ce que la ou les chaînes R comprennent un polyoxyde d'éthylène de formule (A) lié de façon covalente auxdites unités monomères via un pont hydrolysable.

De tels matériaux sont particulièrement avantageux dans la mesure où ils permettent une libération contrôlée de principes actifs stables ou pas *in vivo.* Suivant cette stratégie la libération du principe actif, emprisonné à l'intérieur de la particule, donc isolé du milieu extérieur, et lié de manière covalente à celle-ci, se fait par une première étape de déstabilisation desdites particules par rupture des liaisons entre les unités monomères et les chaînes R via un stimuli extérieur (tel que pH, hyperthermie...), ce qui entraîne un relargage des chaînes R stabilisantes. Les chaînes résultantes, R ou Z1, fonctionnalisées ou non par le principe actif subissent dans un deuxième temps des réactions d'hydrolyses et libèrent le principe actif.

Les matériaux de l'invention sont des matériaux à la surface desquels sont liées des particules sphériques stimulables, c'est-à-dire sensibles à un stimuli extérieur tel qu'une variation de pH ou de température, ce qui permet alors la libération des principes actifs emprisonnés à l'intérieur de ces particules.

De préférence les ponts hydrolysables susmentionnés sont choisis parmi les enchaînements d'environ 1 à 10 motifs d'ε-caprolactone, ou de fonctions -OC(O)-,

-C(O)OC(O)-, -C(O)-NH-...

A ce titre, l'invention a plus particulièrement pour objet des biomatériaux tels que définis ci-dessus, caractérisés en ce que la ou les chaînes R comprenant un polyoxyde d'éthylène de formule (A) lié de façon covalente à un pont hydrolysable choisi parmi les enchaînements d'environ 1 à 10 motifs d'ε-caprolactone, sont représentées par la formule

-CH₂-(O-CO-(CH₂)₅)ₜ-O-CO-(CH₂)₅-O-CO-(CH₂)₂-CO-O-(CH₂-CH₂-O)ₙ-(CH₂)₂-O-X

dans laquelle t représente un nombre entier compris entre 1 et 10, et X représente H, -CH₂-COOH, -CH₂-COCl, ou -CH₂-COY, Y représentant un principe actif, ou une molécule biologique telle qu'une protéine.

Avantageusement, les biomatériaux de l'invention tels que définis ci-dessus sont caractérisés en ce que le matériau support est choisi parmi :
- les métaux, tels que le Titane,
- les alliages métalliques, notamment les alliages à mémoire de forme ou non tels que les alliages Ni-Ti
- les polymères, tels que le polyéthylène téréphtalate (PET), polytétrafluoroéthylène (PTFE), polyfuorure de vinylidène (PVDF), polyéther éthercétone (PEEK),
- les copolymères, tels que le copolymère éthylène-acétate de vinyle (EVA), le copolymère fluorure de vinylidène- hexafluoropropylène P(VDF-HFP), le poly(acide lactique)-co-poly(acide glycolique) (PLA-PGA)
- les céramiques, tels que les hydroxyapatites, ou composées d'hydroxyapatites et de phosphate tricalcique dans des proportions variées, notamment dans les proportions 50/50.

L'invention a également pour objet les biomatériaux tels que définis ci-dessus, caractérisés en ce que la fonction réactive située sur le matériau support afin d'assurer la liaison covalente entre ledit matériau et lesdites particules en réagissant avec la fonction réactive de ces dernières, est du type OH, halogène, NH₂, C(O)X'₁ dans laquelle X'₁ représente un atome d'hydrogène, d'halogène, un groupe OR" ou NHR" dans lequel R" représente un atome d'hydrogène ou une chaîne hydrocarbonée d'environ 1 à 10 atomes de carbone, substituée ou non, pour former une liaison de type -O-C(O)-, -NH-C(O)-, -C(O)-NH-, -C(O)O-, ou -C(OC)₂-, avec la fonction réactive desdites particules.

L'invention a plus particulièrement pour objet les biomatériaux tels que définis ci-dessus, caractérisés en ce que la fonction réactive du matériau support est située sur une chaîne alkyle d'environ 1 à 10 atomes de carbone, greffée sur ledit matériau, substituée ou non, et comprenant le cas échéant un ou plusieurs hétéroatomes, notamment O, et Si, dans ladite chaîne.

L'invention concerne plus particulièrement les biomatériaux tels que définis ci-dessus, caractérisés en ce que :
- la fonction réactive du matériau est une fonction NH₂ située sur une molécule d'aminopropyltriéthoxysilane greffée sur le matériau conformément aux formules suivantes : 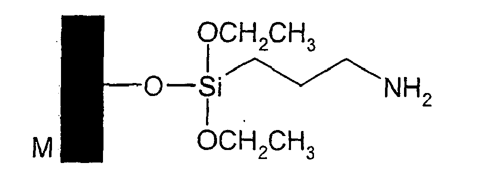 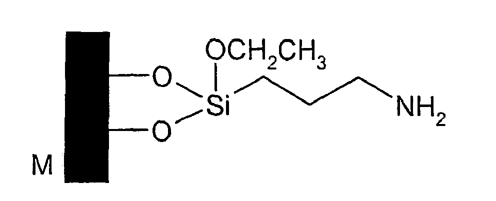 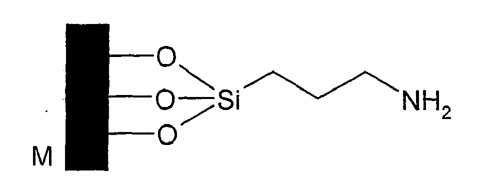 dans lesquelles M représente un oxyde métallique, ou une céramique telle que l'hydroxyapatite, ou tout autre polymère comportant des sites OH en leur surface (par nature ou par le biais d'une préfonctionnalisation),
- la fonction réactive du matériau est une fonction NH₂ située sur une surface préfonctionnalisée par de l'acide acrylique laquelle est couplée à un bras espaceur bifonctionnel tel que le bNH₂PEG (O, O'-bis-(2-aminopropyl)-polyéthylène glycol 500. (cette préfonctionnalisation est décrite dans l'article Nucl. Instr. And Meth. In Phys. Res. B 151 1999 255-262).

L'invention concerne également des biomatériaux tels que définis ci-dessus, caractérisés en ce que le principe actif est choisi parmi les molécules utilisées en thérapeutique, cosmétique, parfumerie, ou pour les revêtements de surface en vue de les rendre incolonisables par différents types de microorganismes : algues, champignons, bactéries,...), tels que les peintures et les antifoulings.

L'invention a plus particulièrement pour objet des biomatériaux tels que définis ci-dessus, caractérisés en ce que le principe actif est un médicament utilisé en thérapeutique choisi notamment parmi ceux des classes thérapeutiques suivantes : antibiotique, anti-inflammatoire, antimitotique, hormones, facteurs de croissance.

L'invention concerne également l'utilisation de biomatériaux tels que définis ci-dessus, pour la préparation de dispositifs médicaux implantables, notamment sous forme d'implants, de prothèses, de stents, ou de ciments, notamment en chirurgie vasculaire, endovasculaire, ou osseuse.

L'invention a également pour objet les implants, prothèses, stents vasculaires, ou ciments, ainsi que toute composition pharmaceutique comprenant des biomatériaux tels que définis ci-dessus.

Les molécules utilisées en thérapeutique, cosmétique, parfumerie, ou pour les revêtements de surface en vue de les rendre incolonisables par différents types de microorganismes : algues, champignons, bactéries,...), tels que les peintures et les antifoulings.

L'invention concerne plus particulièrement des biomatériaux tels que définis ci-dessus, caractérisés en ce que la molécule biologique est choisie parmi les protéines susceptibles de se lier à une cible biologique intra ou extracellulaire, ou à des anticorps, ou à tout autre ligand spécifique.

L'invention a plus particulièrement pour objet des biomatériaux tels que définis ci-dessus, caractérisés en ce que la molécule biologique est choisie parmi les protéines suivantes : l'avidine, l'albumine, les facteurs de croissance tel que le VEGF.

L'invention concerne également des compositions pharmaceutiques comprenant des biomatériaux tels que définis ci-dessus dans lesquels le ou les différents groupes X contiennent un principe actif médicamenteux, le cas échéant en association avec un véhicule pharmaceutiquement acceptable.

L'invention a également pour objet des compositions cosmétiques comprenant des biomatériaux tels que définis ci-dessus, dans lesquels le ou les différents groupes X contiennent un principe actif utilisé en cosmétique, le cas échéant en association avec un véhicule approprié, notamment pour une application sous forme d'émulsions, crèmes.

L'invention concerne également des compositions de revêtement de surfaces comprenant des particules sphériques telles que définies ci-dessus, dans lesquelles le ou les différents groupes X contiennent un principe actif utilisé pour les revêtements de surfaces, le cas échéant en association avec un véhicule approprié.

L'invention a également pour objet un procédé de préparation de biomatériaux de l'invention caractérisé en ce qu'il comprend :
- une étape de polymérisation d'un alcène mono ou polycyclique tel que défini ci-dessus substitué par une chaîne R telle que définie ci-dessus, le cas échéant en présence:
   * d'un ou plusieurs alcènes mono ou polycycliques tels que définis ci-dessus, identiques ou différents du précédent, et substitués par une chaîne R telle que définie ci-dessus, ladite chaîne R étant différente de celle substituant l'alcène mono ou polycyclique susmentionné,
   * et/ou d'un ou plusieurs alcènes mono ou polycycliques tels que définis ci-dessus, identiques ou différents du précédent, et substitués par un groupe X tel que défini ci-dessus, ce groupe X étant identique ou différent du groupe X de la chaîne R des alcènes précédents,
   * et/ou d'un ou plusieurs alcènes mono ou polycycliques tels que définis ci-dessus, identiques ou différents des précédents, lesdits alcènes n'étant pas substitués, ladite polymérisation étant effectuée sous agitation en présence d'un complexe de métal de transition en tant qu'amorceur de la réaction choisi notamment parmi ceux des groupes IV ou VI ou VIII, tel que le ruthénium, l'osmium, le molybdène, le tungstène, l'iridium, le titane, en milieu polaire ou apolaire, notamment à l'aide des complexes à base de ruthénium suivants : RuCl₃, RuCl₂(PCy₃)₂CHPh,...
- et une étape de fixation desdites particules sphériques obtenues à l'étape précédente, sur un matériau support tel que défini ci-dessus, par mise en présence desdites particules avec ledit matériau, ce dernier ayant été le cas échéant fonctionnalisé avec une fonction réactive telle que définie ci-dessus capable d'assurer la liaison covalente entre ledit matériau et lesdites particules en réagissant avec la fonction réactive de ces dernières.

L'invention sera davantage illustrée à l'aide de la description détaillée qui suit de l'obtention de biomatériaux de l'invention, et de leurs caractéristiques physicochimiques.

La synthèse des particules sphérique se fait en une étape et permet de moduler facilement la cinétique de libération de molécules actives en fonction de l'application envisagée. En outre, le fait de pouvoir greffer cet objet de façon covalente sur le matériau lui confère d'excellentes propriétés de tenue mécanique et lui assure une stabilité dans le temps.

L'utilisation des particules sphériques permet à la fois d'accroître la surface spécifique du matériau afin de garantir une concentration suffisante en molécules bioactives et d'introduire facilement plusieurs fonctions chimiques ou principes actifs à la surface du biomatériau.

Schématiquement, l'élaboration du dispositif proposé peut se découper en 3 étapes distinctes :
1-La fonctionnalisation du biomatériau
2-La synthèse des nanoparticules bioactives
3-La fixation des nanoparticules sur le biomatériau

### 1 - La fonctionnalisation du biomatériau

En terme de matériaux, le développement d'une prothèse bioactive nécessite le contrôle des interfaces matériaux-molécules ou matériaux-biomolécules. Le greffage est une technique permettant de fixer par liaison covalente à la surface de tout type de matériau une ou plusieurs molécules choisies pour leurs propriétés spécifiques. La totalité du traitement est réalisé sous atmosphère, température et pression contrôlées ce qui permet une parfaite maîtrise des conditions de greffage. La technique employée consiste en une modification de la fonctionnalité en surface du biomatériau afin de la rendre plus réactive.

A titre illustratif, les conditions expérimentales utilisées dans la cas du greffage d'une molécule d'aminopropyltriéthoxysilane (APTES) sur hydroxyapatite (HA) (schéma 1) ont été reportées ci-dessous

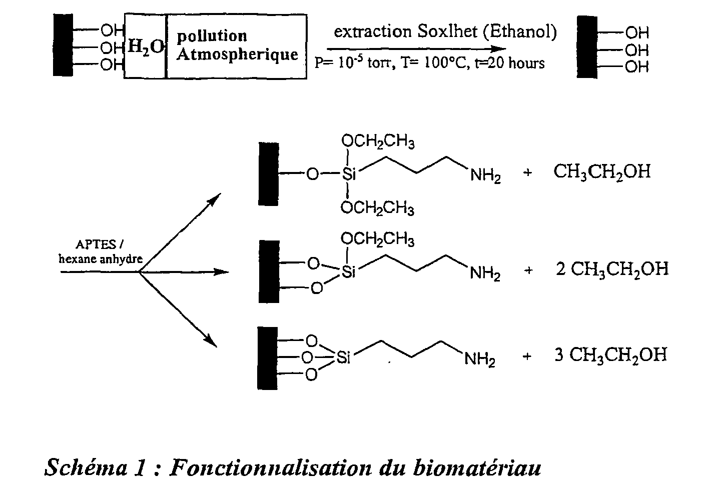

### a) Préparation de la surface

L'HA a été lavée à l'aide d'un extracteur Soxlhet (à éthanol) pendant 24 h.

### b) Modification de la surface

La modification de la surface a été effectuée dans une chambre sèche et dépourvue d'air afin d'éviter une contamination de la surface par de l'eau et des composés carbonés provenant de l'atmosphère environnant et ce afin de s'assurer de la reproductibilité et de la stabilité de la couche moléculaire. La stratégie d'immobilisation du ligand (schéma 1) implique le greffage d'un organosilane aminofonctionnel (APTES) sur la surface d'hydroxyapatite (HA).

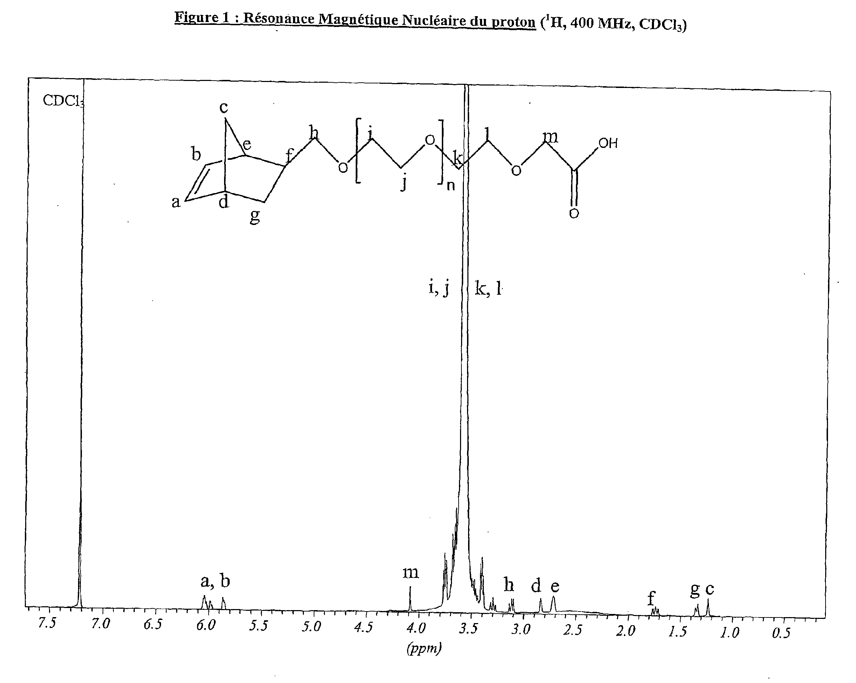

Expérimentalement, la modification de la surface d'HA a été réalisée par la procédure suivante, également représentée dans la Fig. 1.
1. L'HA a été dégazée à 100°C sous vide (10⁻⁵ torr) pendant 20 h (surface A).
2. La silanisation de la surface d'HA a été réalisée en immergeant le substrat dans une solution d'APTES (1.10⁻² M) dans de l'hexane anhydre sous atmosphère Ar pendant 2 heures sous agitation.
3. Les échantillons ont été lavés sous atmosphère Ar par 3 rinçages sous agitation et sonication pendant 30 min (les deux étapes ont été réalisées en utilisant de l'hexane anhydre).
4. Les échantillons ont été dégazés à 70°C sous vide (10⁻⁵ torr) pendant 4 heures (surface B).

### c) Caractérisation de la surface

La spectroscopie photoélectronique aux rayons X (XPS) a été appliquée au contrôle des réactions à chaque étape de la procédure. Les spectres XPS ont été enregistrés à l'aide d'un spectromètre VG 220i-XL Escalab sur les substrats HA à chaque étape du greffage des peptides RGD. La puissance de la source MgKα non monochromatique était de 200 W avec une zone étudiée d'environ 250 microns. Un canon à électrons a été utilisé pour compenser les charges. L'acquisition de spectres à haute résolution a été faite à passages d'énergie constants de 20 eV. L'ajustement a alors été réalisé à l'aide d'un logiciel fourni par VG Scientific, chaque spectre ayant pour référence une pollution en carbone à 284,8 eV. Les valeurs d'énergie de liaison (BE) sont données à ± 0,2 eV.

### 2 - La synthèse des nanoparticules

La synthèse des nanoparticules est réalisée par copolymérisation en milieu dispersé de monomères vinyliques (cyclo-oléfines) avec des macromonomères α,ω-fonctionnalisés par une entité polymérisable et par une fonction réactive et/ou un principe actif (médicaments, molécules organiques...). Des exemples de cette synthèse sont détaillés ci-après.

### a) synthèse des macromonomères de formules A et B ci-après

Les macromonomères (A et B) sont des oligomères de poly(oxyde d'éthylène) de 7000 g/mol de masse molaire (M̅ₙ). Ils sont issus d'une polymérisation anionique 'vivante' ce qui permet de contrôler la longueur et la fonctionnalité des chaînes. Ils sont fonctionnalisés à l'une de leur extrémité par une unité norbornényle, entité choisie pour sa réactivité élevée en polymérisation par métathèse et, à l'autre extrémité par une fonction réactive de type alcool, acide, amine...(A), ou par le principe actif (indométacine) (B) via un pont clivable (anhydride d'acide, ester, amide, ...).

### a-1. α-norbornényl-ω-carboxylic acid-poly(ethylene oxide) ; formule A

### Formule chimique :

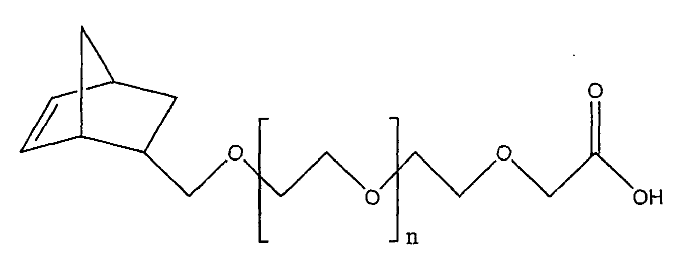

avec n compris entre 50 et 340 en fonction des besoins de l'application envisagée.

### Référence : NB-POE-COOH.

### Schéma de synthèse :

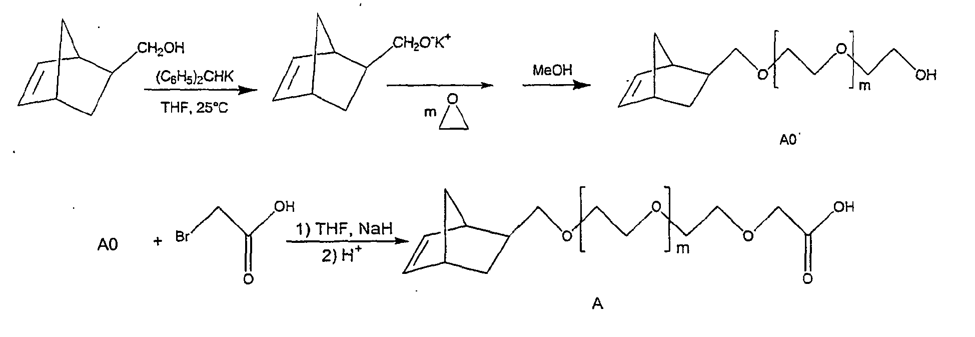

### Procédure de synthèse :

Le 5-norbornène-2-méthanol (0,5 mL) en solution dans le tétrahydrofurane (THF) (200mL) est tout d'abord déprotoné par l'ajout d'un équivalent molaire de diphénylméthyl potassium. L'espèce résultante va ensuite amorcer la polymérisation de l'oxyde d'éthylène (28 mL) de manière « vivante » (48h) jusqu'à la destruction des centres actifs par l'ajout de méthanol (1mL). La fonction alcool du poly(oxyde d'éthylène) obtenu (A0) va alors être transformée en fonction acide par déprotonation de A0 (10g) avec NaH (0,17 g) en solution dans le THF (15mL), suivi de l'ajout d'acide bromoacétique (0,42g). Après lavage du produit à l'acide chlorhydrique (18 mL, 1M) puis précipitation dans l'éther, le macromonomère A est obtenu sous une forme pure.

### a-2. α-norbornenyl-ω-indométacine-poly(ethylene oxide) ; formule B

### Formule chimique :

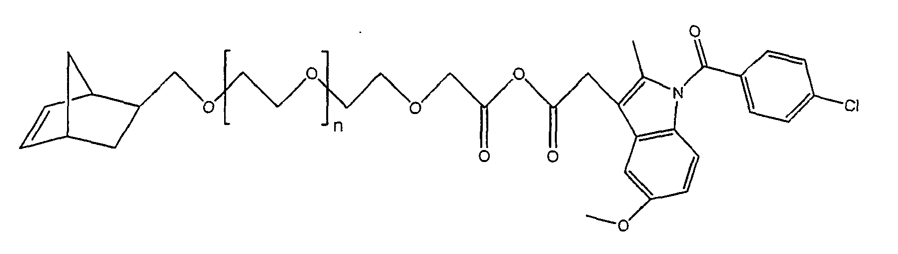

avec n compris entre 70 et 200 en fonction des besoins de l'application envisagée.

### Référence: NB-POE-CO(O)-IND.

### Schéma de synthèse:

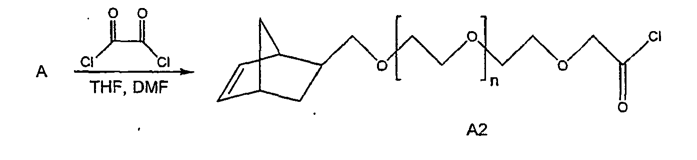

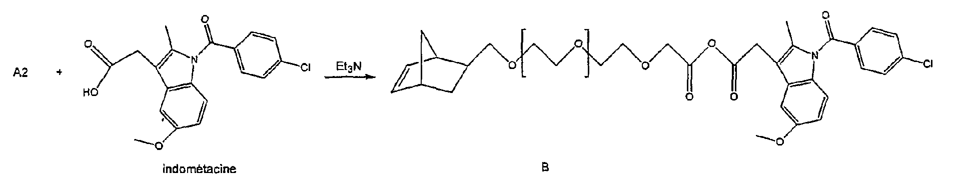

### Procédure de synthèse:

La fonction acide du NB-POE-COOH (A) est transformée en chlorure d'acide (A2) par réaction de A (5,2g) sur le chlorure d'oxalyl (0,08 mL) dans le THF (25mL) en présence d'une quantité catalytique de diméthylformamide pendant 24h. L'indométacine (0,6g) ainsi que la triéthylamine (0,24mL) sont alors additionnés à la solution de A2 et laisser sous agitation pendant 15h. Après précipitation dans l'éther, le macromonomère B est obtenu.

### a-3. Dérivé indométacine du norbornène

Le monomère utilisé dans les réactions précédentes est le norbornène (NBH), ou le norbornène fonctionnalisé (NBD) par le principe actif. Celui-ci est alors introduit via un pont hydrolysable de type ester, anhydride, amide..... La synthèse du norbornène fonctionnalisé par l'indométacine est décrite ci-après.

### Formule chimique :

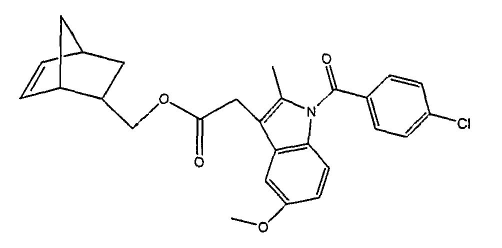

Référence : NBD.

### Schéma de synthèse :

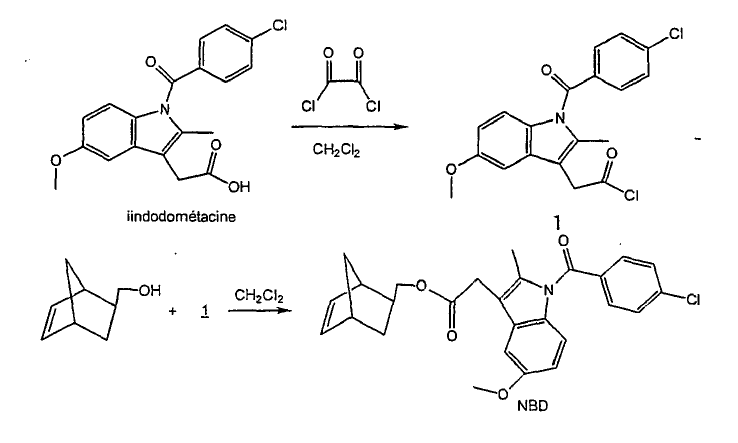

### Synthèse du monomère NBD

Lors d'une réaction type, le chlorure d'oxalyle (0,87mL) est additionné à l'indométacine (1,1g) en solution dans le dichlorométhane (20mL). Après 2 heures de réaction et élimination du chlorure d'oxalyle non réagi, le composé 1 obtenu est ensuite ajouté à une solution de 5-norbornène-2-méthanol (0,36 mL) dans du dichlorométhane (20mL) en présence de triéthylamine (0, 84mL) et laissé sous agitation pendant 15h à 45°C. Après purification par extraction, le monomère NBD est obtenu (p>95%).

### b. Synthèse des particules

Les particules de l'invention sont obtenues par copolymérisation en milieu dispersé (émulsion, mini- et micro-émulsion, dispersion, suspension) de monomères vinyliques (cyclo-oléfines) avec des macromonomères α,ω-fonctionnalisés par une entité polymérisable et une fonction réactive ou un principe actif (médicaments, molécules organiques...). La polymérisation est amorcée par des métaux de transition et peut se faire en milieu aqueux ou organique (dichlorométhane/éthanol). Les macromonomères jouent le rôle de stabilisant et d'agent de fonctionnalisation. En tant que stabilisants ils permettent, lors de la formation du polymère dans le milieu réactionnel, de le disperser sous forme de nanoparticules sphériques. Purement stérique la stabilisation est insensible à toute variation de pH du milieu. Par ailleurs, la fonctionnalisation du latex par l'intermédiaire de macromonomère améliore la disponibilité des fonctions réactives à la surface du latex et préserve leur réactivité.

L'amorceur de la polymérisation est un complexe à base de ruthénium stable en milieu polaire : RuCl ₃, RuCl₂(PCy₃)₂CHPh et leurs homologues. Le latex synthétisé dans ces conditions sera constitué de chaînes de polyalcénamère porteuses de greffons poly(oxyde d'éthylène) qui serviront à stabiliser les particules.

Les particules obtenues sont stables en milieu aqueux ou /et organique. Leur taille est comprise entre quelques nanomètres et quelques micromètres en fonction du procédé de polymérisation (dispersion, suspension, mini-émulsion...) utilisés. Les nanoparticules sont sphériques et de très bonne isométrie .

### Schéma de synthèse :

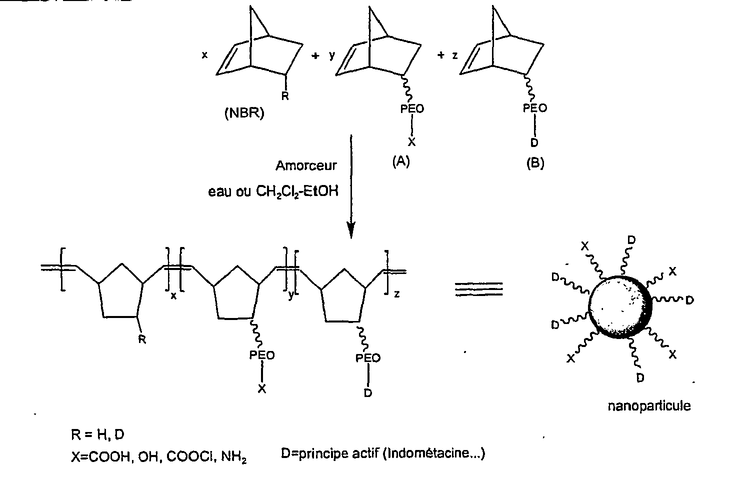

### Procédure de synthèse :

Les macromonomères A et B sont copolymérisés en présence de monomère (NBH et/ou NBD). Dans une réaction type, 0,8g de monomère et 1g de macromonomère (0,2g de A et 0,8g de B) préalablement dissous dans 14ml d'un mélange dichlorométhane/éthanol (35%/65%) sont ajoutés sous azote et sous vive agitation à 10ml de dichlorométhane/éthanol (50%50%) contenant 20mg d'amorceur. La durée de la polymérisation est de une heure. Le milieu totalement homogène au départ devient de plus en plus turbide au fur et à mesure que la polymérisation a lieu. Le suivi des polymérisations, par chromatographie gazeuse, a révélé des conversions des monomères totales en moins de une minute. L'incorporation des macromonomères A et B dans le latex est totale.

### c. Variante pour le transport de principe actif sensible.

Le latex est réalisé comme précédemment par copolymérisation entre une cyclooléfine (le norbornène) porteuse d'un principe actif (Indométacine) ou pas et le stabilisant polymère (NB-PCL-POE-OMe). Ce dernier fonctionnalisé ou pas par une fonction réactive de type acide, chlorure d'acide, alcool, amine (même fonction que précédemment) possède un pont hydrolysable, notamment des motifs d'ε-caprolactone (PCL) entre la fonction polymérisable et la chaîne de polyoxyde d'éthylène selon le schéma ci-après.

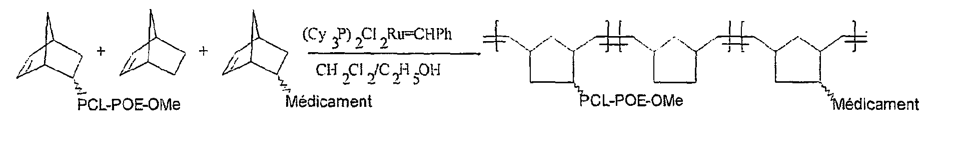

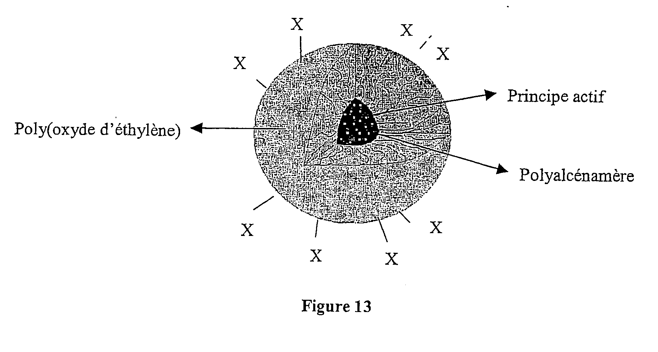

Suivant cette stratégie la libération du principe actif, emprisonné à l'intérieur de la particule et lié de manière covalente à celle-ci (Figure 13), nécessite une première étape de déstabilisation du latex. Celle-ci peut être réalisée via un stimuli extérieur (pH, hypothermie...) par relargage des chaînes stabilisantes.

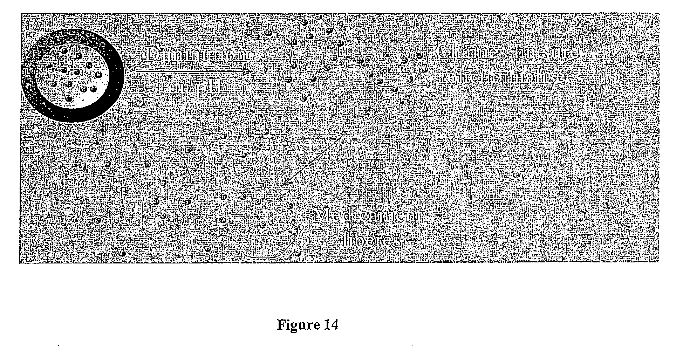

Les chaînes linéaires de polyalcénamères résultantes fonctionnalisées par le principe actif subissent dans un deuxième temps des réactions d'hydrolyses et libèrent le principe actif (Figure 14).

### c-1) Procédure pour la synthèse copolymère poly(caprolactone-b-éthylène glycol)-a-norbomène-ω-méthyléther NB-PCL-POE-OMe

### Préparation du poly(caprolactone) α-norbornènyle (NB-Pcapro)

A une solution de 2-hydroxyméthyl-5-norbornène (1,3.10⁻² mole) dans le toluène (100 mL) refroidie à -80°C est ajouté le triéthylaluminium (1,3.10⁻² mole) goutte à goutte. Après un retour progressif à température ambiante, la réaction est poursuivie pendant 2,5 heures. La caprolactone (3,9 mole) est ensuite additionnée au milieu réactionnel sous agitation vigoureuse. Après 18 heures de réaction, 50 mL d'acide chlorhydrique (0,1 N) sont ajoutés. Après un lavage à neutralité, le poly(ε-caprolactone)-α-norbornènyle est précipité à froid dans l'heptane puis filtré sur fritté n°4. Les traces d'heptane seront éliminés par un chauffage (40°C) sous vide pendant 10 heures. Le polymère obtenu est ensuite lyophilisé 3 fois avec du dioxane en tant que solvant.

### Préparation du poly(éthylène glycol)-α-acide carboxylique-ω-méthyléther

Solubiliser 3,89.10⁻³ mole d'anhydride succinique et 4.10⁻³ mole de triéthylamine dans 45 mL d'acétone anhydre. Ajouter sous agitation et goutte à goutte une solution de poly(éthylène glycol) monométhyléther (6.10⁻⁴ mole) dans 15 mL de CH₂Cl₂ anhydre. Après 16 heures de réaction, additionner 1 mL de méthanol. Après concentration à l'évaporateur rotatif, précipiter le polymère dans l'éther éthylique. Recommencer 2 autres fois les étapes de dissolution/précipitation. Placer le polymère sous vide dynamique pendant 10 heures pour éliminer toutes traces de solvant.

### Préparation du copolymère poly(caprolactone-β-éthylène glycol)-α-norbornène-ω-méthyléther (NB-Pcapro-PEG-OMe)

Solubiliser 4.10⁻⁴ mole de poly(éthylène glycol)-α-acide carboxylique-ω-méthyléther dans 40 mL de CH₂Cl₂ anhydre. Ajouter à cette solution refroidie à 5°C le chlorure d'oxalyle (8.10⁻⁴ mole). Après 15 heures de réaction, enlever l'excès de chlorure d'oxalyle non réagi ainsi que le CH₂Cl₂ sous pression réduite. Le résidu jaune obtenu est ensuite redissous dans 40 mL de dichlorométhane. Après avoir additionné la triéthylamine (4,3.10⁻⁴ mole), ajouter goutte à goutte et sous agitation le poly(caprolactone) α-norbornènyle. Après concentration à l'évaporateur rotatif, précipiter le polymère dans l'éther éthylique. Recommencer 2 autres fois les étapes de dissolution/précipitation. Placer le polymère sous pression réduite pendant 10 heures pour éliminer toutes traces de solvant.

La synthèse du poly(ε-caprolactone)α-norbornényle est effectuée selon le schéma suivant :

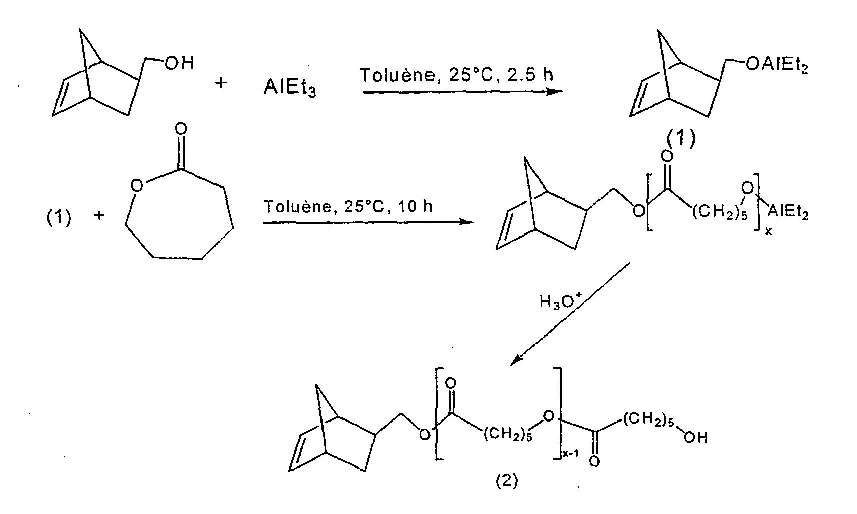

La synthèse du poly(ε-caprolactone-b-éthylène glycol)-α-norbornène-ω-méthyléther est effectuée selon le schéma suivant :

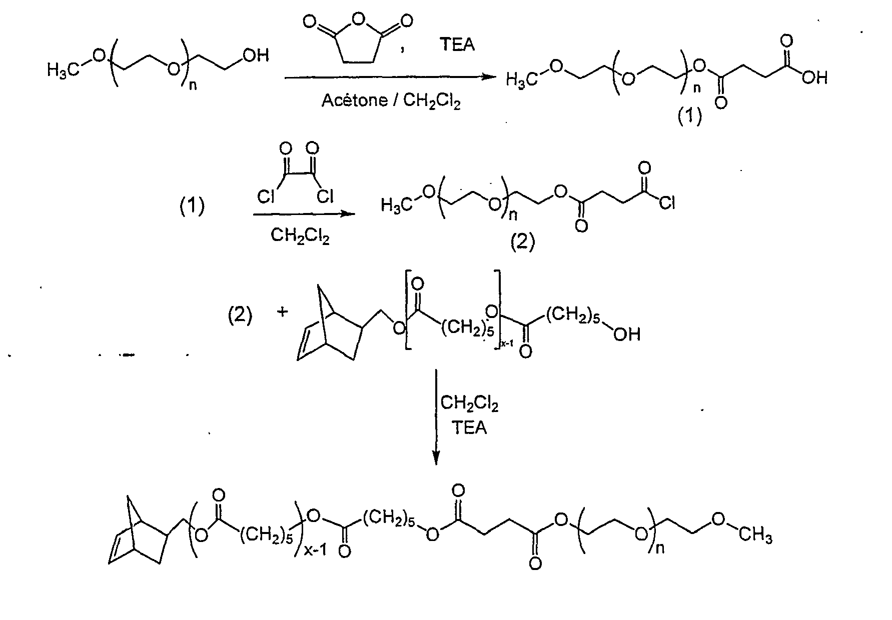

### c-2) Etude de la libération du principe actif

Une fois la particule synthétisée, nous avons vérifiés par spectrométrie UV-Visible la possibilité de libérer le médicament par simple abaissement du pH. Les résultats obtenus ont permis de confirmer une libération progressive et contrôlée de l'indométacine. Par ailleurs, l'application d'un pH égal à 3 a révélé que plus de 85% de celui-ci pouvait être relargué en 48h

### 3 - La fixation des nanoparticules sur le biomatériau

La fixation covalente des particules sur le matériau est réalisée par condensation de deux fonctions réactives antagonistes, se trouvant l'une sur le matériau et l'autre sur les particules. On peut citer à titre d'exemple les couples acide/amine, acide/alcool, acide/chlorure d'acide, alcool/chlorure d'acide ....

Dans le cas qui nous intéresse cette réaction est effectuée entre un matériau comportant des fonctions réactives (ici des amines) et des nanoparticules fonctionnalisées à la fois par des molécules bioactives (dans notre exemple par l'indométacine) et des sites d'ancrage (ici des acides). Le matériau est alors dit bioactif, c'est à dire qu'il possède une activité biologique (schéma 2). Dans notre exemple, cette activité est stimulée par la libération du principe actif, sous sa forme native, lors du contact du matériau avec le milieu physiologique ou par une modification du pH. Pour cela une liaison clivable est introduite entre la nanoparticule et le principe actif

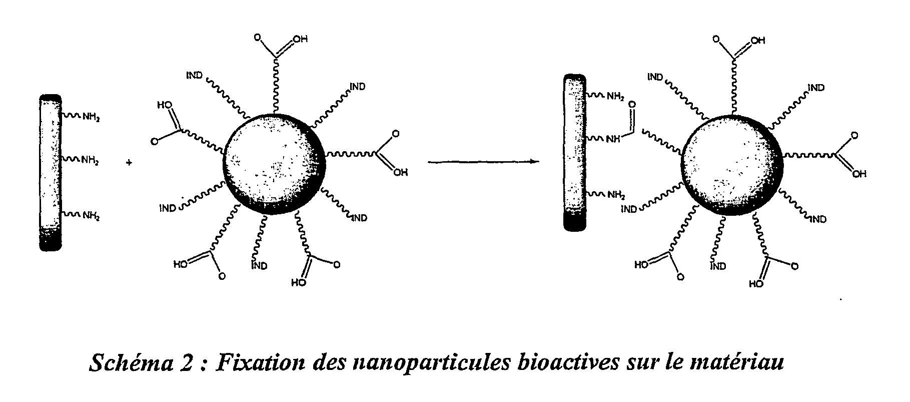

Lors d'une réaction type, 3.10⁻⁴ mol d'hydroxybenzotriazole (HOBT) est ajoutée à une solution de nanoparticules (3,66.10⁻⁵ mol de fonctions acide) dans 2 mL de diméthylformamide. Après une complète solubilisation de l'HOBT, le matériau est alors introduit puis laissé sous agitation pendant 15 min à température ambiante. 2,5.10⁻⁴ mol de 1-(3-diméthylaminopropyl)-3-éthylcarbodiimide est ensuite additionné au milieu réactionnel qui est maintenu pendant 12 heures sous agitation. Le matériau fonctionnalisé par des particules bioactives est ensuite purifié par lavages successifs puis séché.

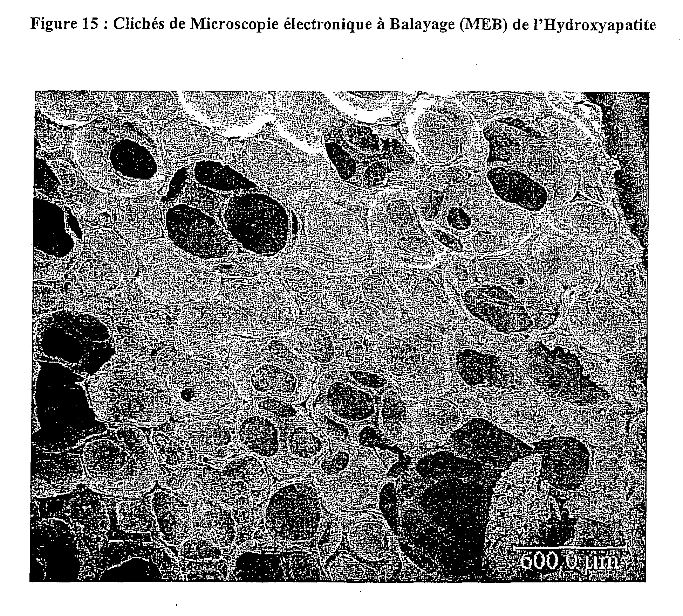

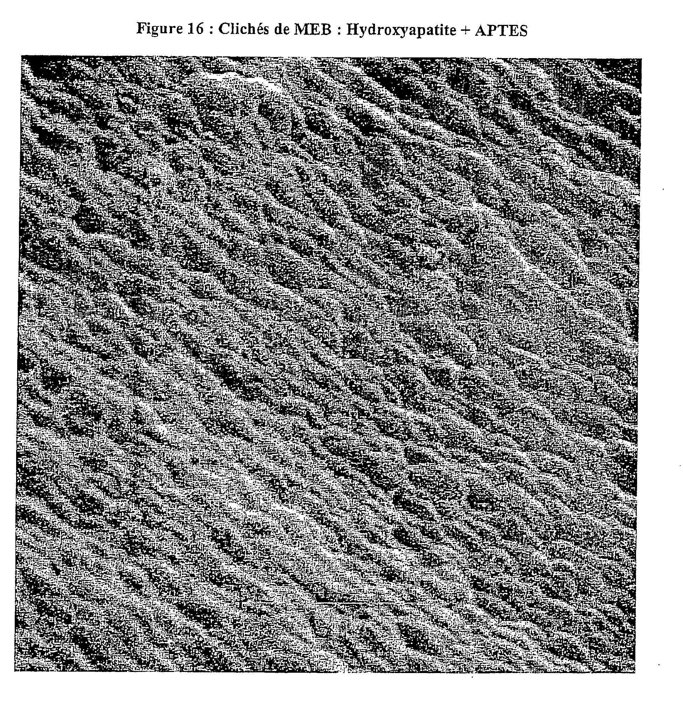

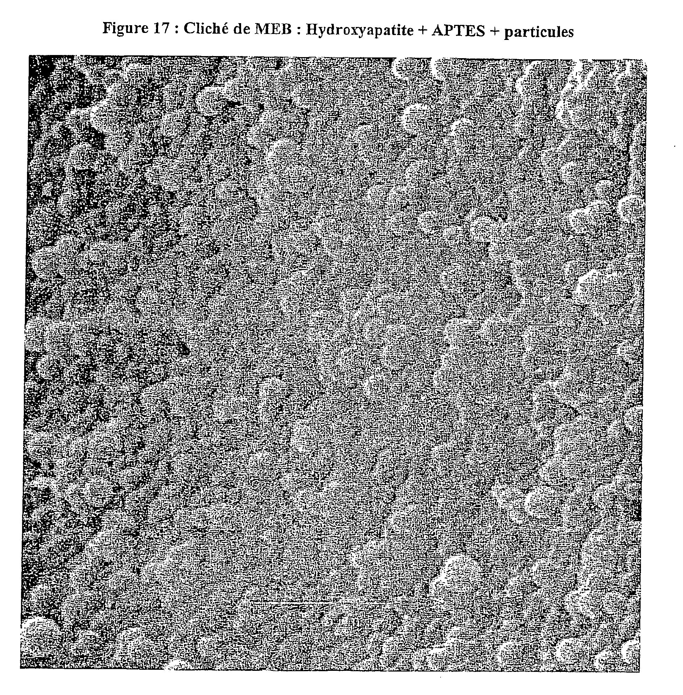

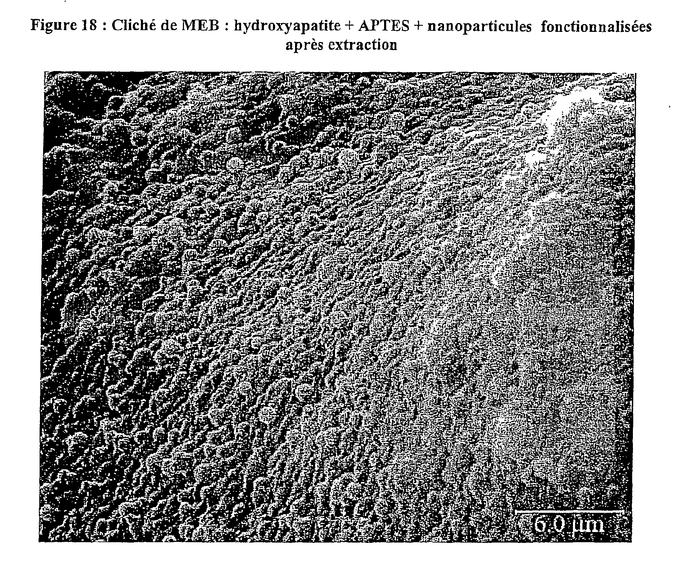

La topographie des matériaux d'hydroxyapatite a été étudiée par Microscopie Electronique à Balayage (MEB) (Figures 15-17 ; grandissement : 20 000 ; Figures 18-19 ; grandissement : 5 000). La présence de nanoparticules a été clairement mise en évidence par comparaison des topologies des matériaux avant (clichés des figures 15, 16) et après (cliché de la figure 17) la réaction de greffage.

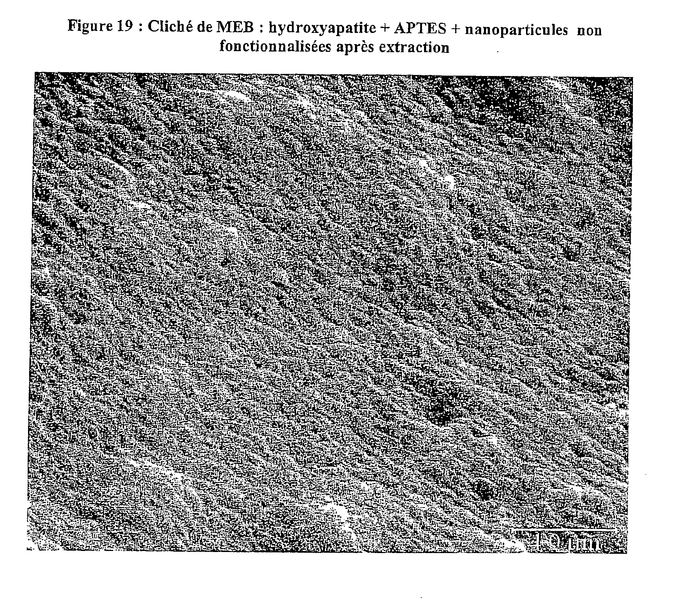

Par ailleurs, la stabilité de l'ancrage chimique des nanoparticules a été vérifiée : aucune nanoparticule n'a été libérée suite à une extraction au Soxlhet à l'éthanol pendant 12 h à 80°C (cliché de la figure 18). Le même traitement, appliquée à un matériau comportant des particules simplement physisorbées conduit à l'extraction totale des nanoparticules(cliché de la figure 19).

### Procédure de biofonctionnalisation

La XPS a été utilisée pour suivre chaque étape de la réaction car elle peut fournir des informations sur les liaisons chimiques et les concentrations atomiques. Le Tableau 1 donne les modifications des proportions atomiques sur les surfaces supérieures.

**Tableau 1 : Composition atomique par XPS en pourcentage d'HA à chaque étape du traitement**

| | **Ca** | **P** | **C** | **O** | **Si** | **N** | **N/Si** |
|---|---|---|---|---|---|---|---|
| **HA** | 13.3 | 7.4 | 30.0 | 49.3 | - | - | - |
| **HA + APTES** | 7.0 | 5.5 | 45.5 | 34.0 | 4.0 | 4.0 | 1 |

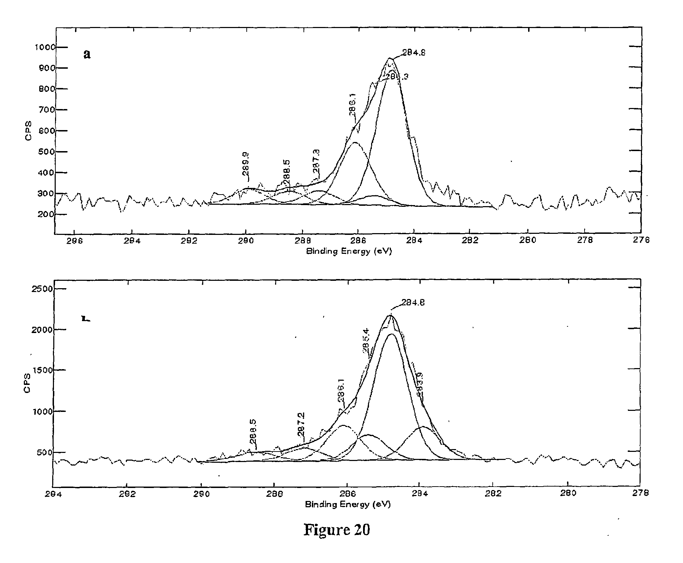

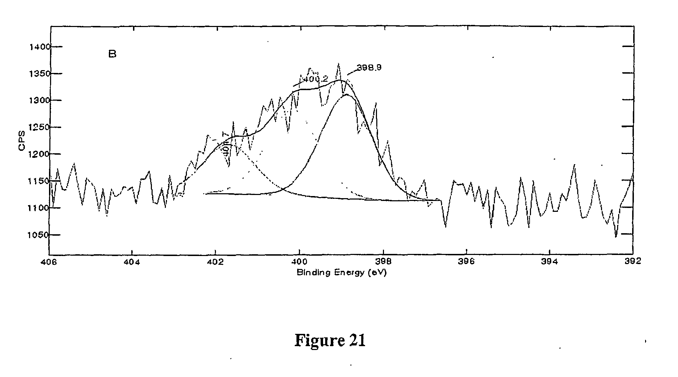

Les Fig. 20 et 21 représentent successivement les spectres C1 et N1 obtenus après chaque étape du traitement. Les énergies de liaison (BE) mentionnées sont comparables avec les BE trouvées dans la littérature (Tableau 2). Dans la discussion suivante, seule la caractérisation en XPS du peptide cyclo-DfKRG est présentée, la procédure de greffage de GRGDSPC conduisant à des résultats semblables.

**Tableau 2: Energies de liaison (eV) assignées à des groupes fonctionnels spécifiques contenant de l'azote, du carbone et du silicium conformément aux données de la littérature.**

| | **C1s** | **Si2p** | **N1s** |
|---|---|---|---|
| **SiO3C** | 283.9 | 102.7 [1] | |
| **CHx** | 284.8 [2] | | |
| **C-CO** | 285.3 [3] [2] | | |
| **C-NH2, C-O** | 285.9-286.1 [2] | | 398.9 [4] |
| **N-C=O** | 286.4 [2] | | 399.7- 400.8 [3] |
| **Imide, Maleimide, guanidine** | 287.2-288 [2] | | 399.7-400.7 [5,6] |
| **COOH** | 288.5 [2] | | |

Les principaux éléments présents dans les surfaces d'hydroxyapatite sont Ca, P, C et O. Le rapport théorique Ca:P attendu est d'environ 1,7. Les rapports expérimentaux obtenus sont successivement 1,8, 1,3, pour HA et HA + APTES, respectivement. Après le greffage de la surface, les détections de Ca et P sont plus difficiles car les électrons doivent parvenir à traverser la couche greffée.

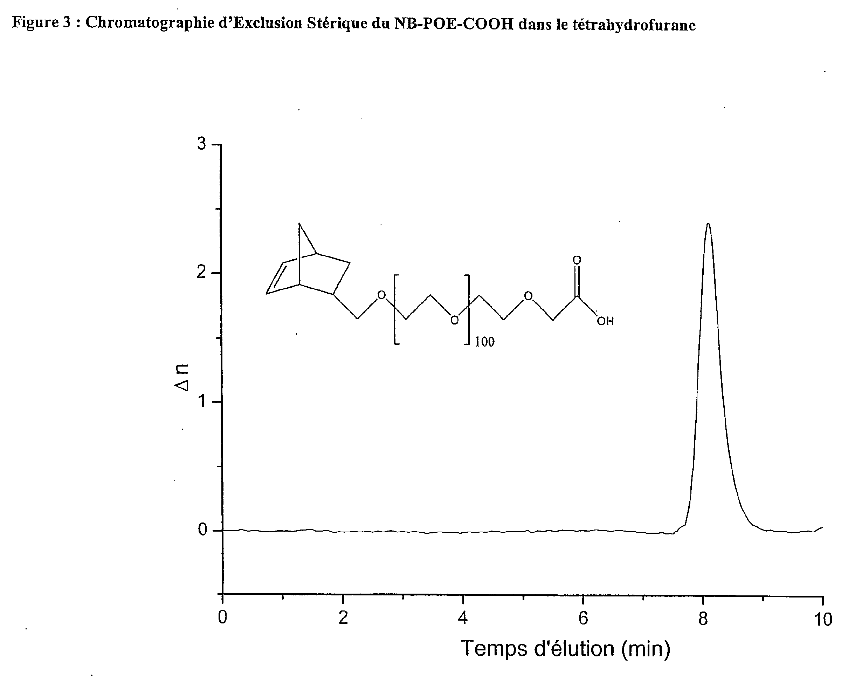

L'analyse en XPS d'HA traitée par de l'APTES confirme que du silicium et de l'azote sont détectés en plus de Ca, P, O et C que l'on trouve habituellement sur les surfaces d'HA. Nos mesures expérimentales (Tableau 2) conduisent à un rapport N:Si proche du rapport attendu théoriquement (4,0:4,0). En plus de liaisons CHx visibles à 284,8 eV (Fig. 20b), des contributions du carbone avec des composants oxydés de plus petite taille (à 285,4 eV) peuvent correspondre à des groupes éthoxy résiduels appartenant à des molécules de silane. Par comparaison au spectre « A » des C1 (Fig. 20a), une nouvelle contribution est visible à 283,9 eV. Ce dernier pic est attribué à des groupes SiO3C (Tableau 2) en accord avec le pic apparaissant à 102,5 eV dans le spectre Si2p. Dans le même temps, le spectre des N1 (Fig. 3a) révèle deux composantes : l'une de basse énergie, caractéristique de groupes C-NH₂ (398,9 eV) et deux autres (à 401,7 eV et 400,2 eV) qui peuvent être attribuées à de l'azote impliqué dans des environnements oxydés. Cette dernière contribution peut être due à certaines interactions entre le groupe aminé terminal et le groupe oxygéné à proximité de la surface. A partir de toutes ces observations, il est évident que les chaînes -CH₂-CH₂-NH₂ sont bien greffées sur la surface.

### 4 - Etude de la libération du principe actif

Après greffage de la nanoparticule sur le matériau, la libération du principe actif (ici indométacine) par contact du matériau avec le milieu physiologique a été réalisée. Les résultats obtenus ont permis de confirmer le relargage contrôlé du principe actif sans altération de la surface du matériau

### 5 - Cytocompatibilité des extraits

La toxicité éventuelle d'un matériau envers des cellules peut être recherchée en étudiant l'effet provoqué par l'extrait de ce matériau. Ces extraits permettent de mettre en évidence l'effet toxique de substances entraînables ou de produits de relargage solubles.

### a) Obtention des extraits

Conformément à la norme, l'obtention des extraits a été réalisée en respectant un rapport surface apparente de la partie immergée de l'échantillon sur le volume du véhicule d'extraction compris entre 3 et 6 cm²/ml. Nous avons choisi de fixer ce rapport à 5 cm²/ml.

Le véhicule d'extraction reste au choix de l'expérimentateur : milieu de culture avec ou sans sérum, solution NaCl 9 ‰ ou tout autre solvant approprié. Nous avons choisi le milieu de culture.

L'extraction est réalisée dans des tubes en verre borosilicaté pour éviter toute interaction. La durée de cette extraction est de 120 heures en incubateur à 37 °C. Au terme de cette période d'extraction, les fragments de matériau sont retirés et le liquide obtenu correspond aux extraits qui seront utilisés au cours des tests.

Nous avons utilisé l'extrait pur (non dilué) ou dilué en utilisant le milieu de culture comme diluant pour obtenir les dilutions de 50 % (v/v), 10 % (v/v) et 1 % (v/v). Une solution de phénol (64 g/l dans le milieu de culture) est inséré comme "témoin positif", c'est-à-dire susceptible d'induire une réponse cytotoxique de façon reproductible.

### b) Ensemencement des cellules et mise en présence des extraits et des solutions

Pour les tests de biocompatibilité, les cellules sont placées dans des plaques de culture de 96 puits (Nunc) qui permettent la lecture sur un spectrophotomètre (Laboratoire Dynatech, Saint-Cloud, France). La densité d'ensemencement est de 6000 cellules/cm² pour les cellules ostéoprogénitrices humaines. En 72 heures, le tapis cellulaire est à subconfluence permettant la réalisation des tests.

### c) Réalisation des tests

Il s'agit de tests colorimétriques permettant de mettre en évidence une activité métabolique cellulaire ou simplement la viabilité cellulaire. La mesure de l'intensité de la réaction colorée à l'aide du spectrophotomètre permet une appréciation quantitative.

### c-1) Test du rouge neutre

### c-1-1)Principe

Ce test a été mis au point par Parish et Mullbacher en 1983 pour déterminer la viabilité cellulaire. Le rouge neutre est un colorant vital qui se fixe par liaison électrostatique aux sites anioniques des membranes lysosomiales dans les cellules vivantes. Une altération de cette membrane provoque une diminution de la fixation du colorant. L'intensité de la réaction colorée permet d'évaluer le nombre des cellules vivantes après incubation en présence d'un agent toxique.

### c-1-2) Protocole

Les plaques de culture sont retirées de l'incubateur après 24 heures de contact entre le liquide d'extraction ou les solutions et les cellules, chaque puits est rincé au moins deux fois à l'aide de 0,2 ml de tampon phosphate. Une solution de rouge neutre (Sigma) à 0,4 % (v/v) dans le milieu de culture (100 µl/puits) est distribuée dans chacun des puits. Après 3 heures d'incubation à 37 °C, la solution de rouge neutre est enlevée et l'extraction du colorant est réalisée en ajoutant 100 µl/puits d'une solution à 1 % (v/v) dans l'eau d'acide acétique dans 50 % (v/v) d'éthanol.

Les plaques sont agitées pendant cinq minutes. On obtient dans chacun des puits, une coloration d'intensité variable dont l'absorbance est mesurée sur le spectrophotomètre à la longueur d'onde de 540 nm. La coloration s'étend de l'incolore pour les colorations entraînant 100 % de toxicité à une couleur plus ou moins rouge pour le témoin et les extraits peu toxiques.

### c-2) Test au MTT

### c-2-1) Principe

Ce test a été introduit par Mosmann en 1983 pour déterminer l'activité métabolique cellulaire. Le MTT ou bromure de 3 - (4,5 - Diméthiazol - 2yl) - 2,5 - diphényl tetrazolium (Sigma) est un sel de tetrazolium de couleur jaune en solution aqueuse à pH neutre. Il est métabolisé par la succinate déshydrogènase mitochondriale des cellules vivantes en cristaux bleu de formazan. La quantité de formazan générée par les cellules, après incubation en présence d'un agent toxique, donne l'indication du nombre et de l'activité métabolique des cellules vivantes.

### c-2-2) Protocole

Les plaques de culture sont retirées de l'incubateur après 24 heures de contact entre le liquide d'extraction ou les solutions et les cellules, chaque puits est rincé au moins deux fois à l'aide de 0,2 ml de tampon phosphate. Une solution de MTT (0,125 ml à 1 g/ml préparé dans un tampon de Hanks contenant 1 g/l de glucose) est distribuée dans chaque puits. Les plaques sont replacées dans l'incubateur pendant 3 heures afin que la réaction enzymatique attendue survienne. Après élimination du surnageant, les cristaux de formazan formés sont solubilisés par l'addition de 0,1 ml/puits de DMSO (diméthylsulfoxyde, Sigma). La solubilisation des cristaux est instantanée, mais leur coloration n'est stable qu'une heure. Les plaques sont donc rapidement lues dans le spectrophotomètre à la longueur d'onde de 540 nm, ce qui permet d'obtenir une valeur d'absorbance par puits. La coloration s'étend de l'incolore pour les concentrations entraînant 100 % de toxicité à une couleur violacée très foncée pour le témoin et les extraits peu toxiques.

### Bibliographie

[1] G. Josefsson, L. Lindberg, B. Wiklander, «Systemic antibiotics and gentamicin-containing bone cement in the prophylaxis of postoperative infections in total hip arthroplasty », Clin. Orthop. 1981 ; 159 : 194-200.
[2] A.D. Hanssen, D.R. Osmon, C.L. Nelson, "Prevention of deep periprosthetic joint infection", J. Bone Joint Surg. 1996 ; 78-A : 458-471.
[3] C.P. Duncan, B.A. Masri, "The role of antibiotic-loaded cement in the treatment of an infection after hip replacement", J. Bone Surg 1994 ; 76-A : 1742-1751.
[4] D.Neut, H. van de Belt, J.R. van Horn, H.C. van der Mei, H.J. Busscher, «Residual gentamicin-release from antibiotic-loaded polymethylmethacrylate beads after 5 years of implantation", Biomaterials 2003 ; 24 : 1829-1831.

### Légendes des figures

- Figure 1 : Spectre RMN ¹H du macromonomère de formule A.

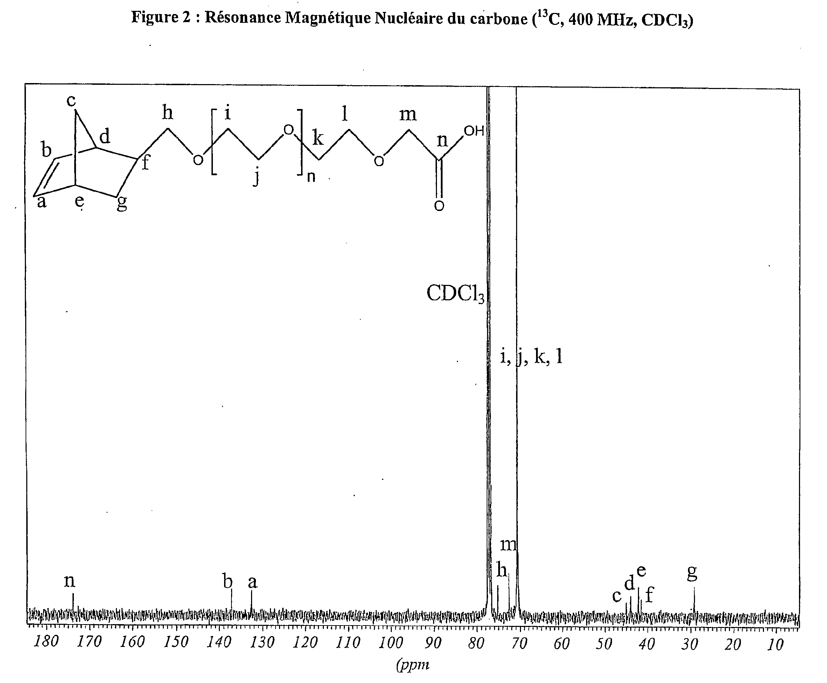

- Figure 2 : Spectre RMN¹³C du macromonomère de formule A.
- Figure 3 :Chromatographie d'exclusion stérique du macromonomère de formule A dans le THF.

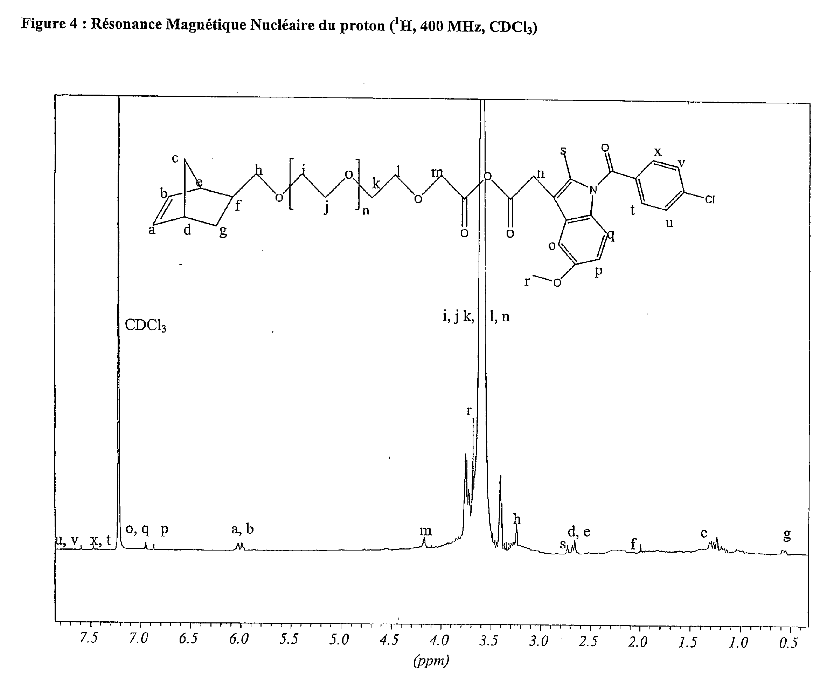

- Figure 4 : Spectre RMN ¹H du macromonomère de formule B.

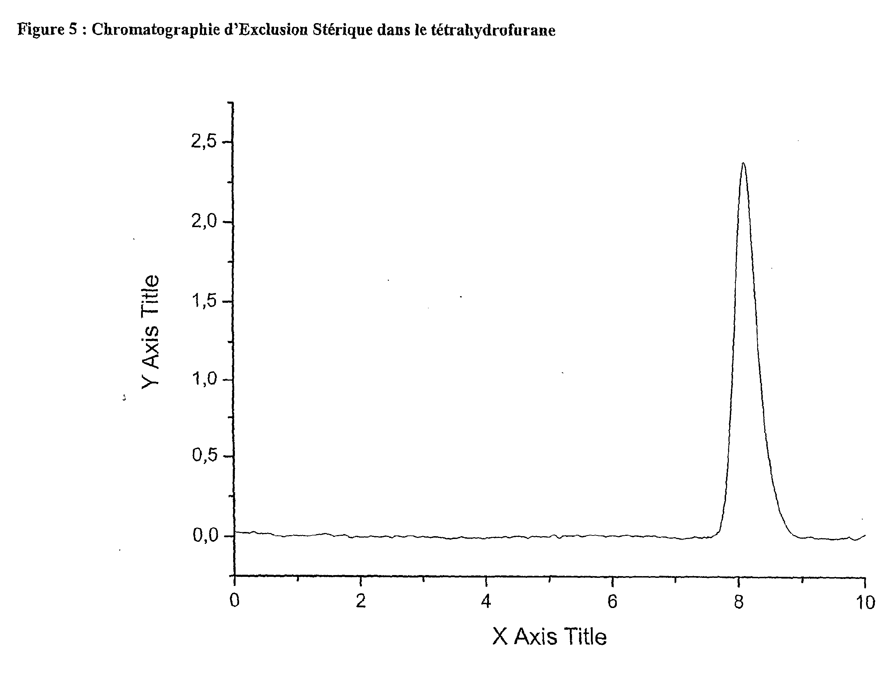

- Figure 5 :Chromatographie d'exclusion stérique du macromonomère de formule B dans le THF.

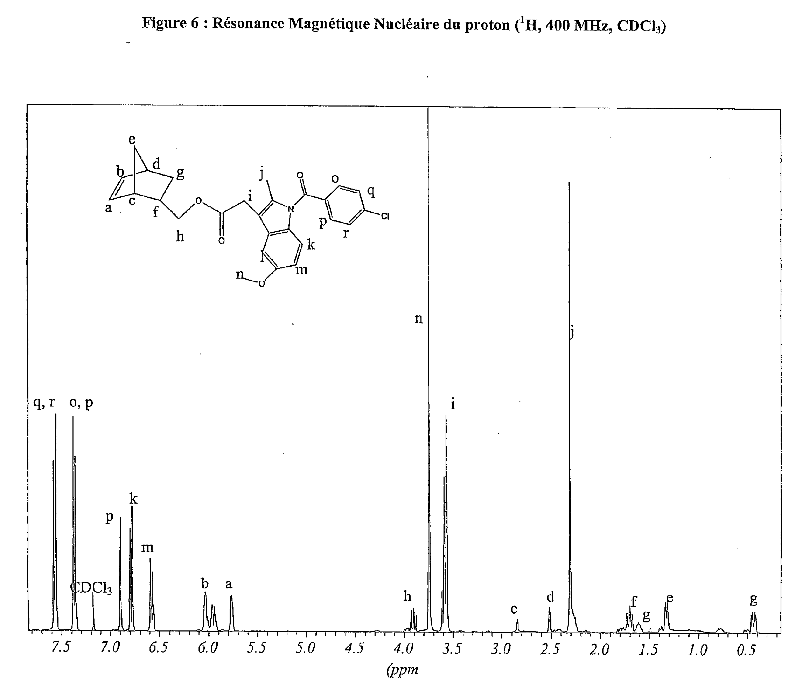

- Figure 6 : Spectre RMN ¹H du composé NBD.

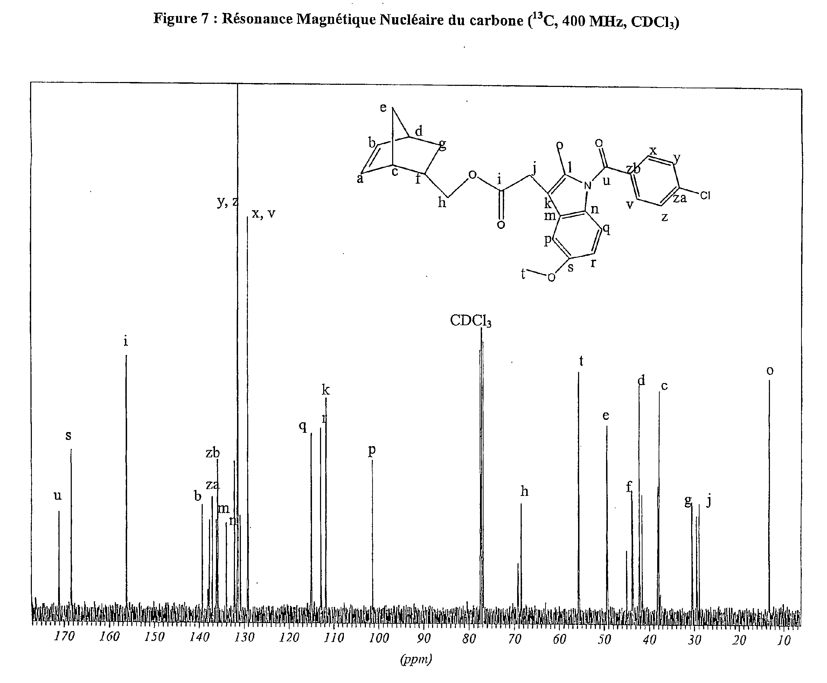

- Figure 7 : Spectre RMN ¹³C du composé NBD.

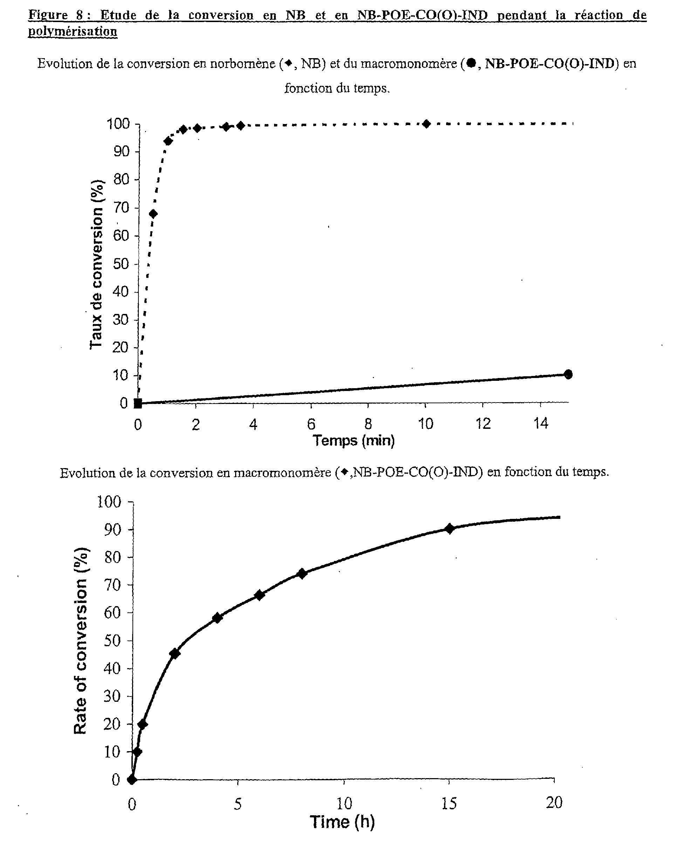

- Figure 8 : Etude de la conversion en NB et en NB-POE-CO(O)-IND pendant la réaction de polymérisation. Evolution de la conversion en norbornène (◆, NB) et du macromonomère (●, NB-POE-CO(O)-IND) en fonction du temps.

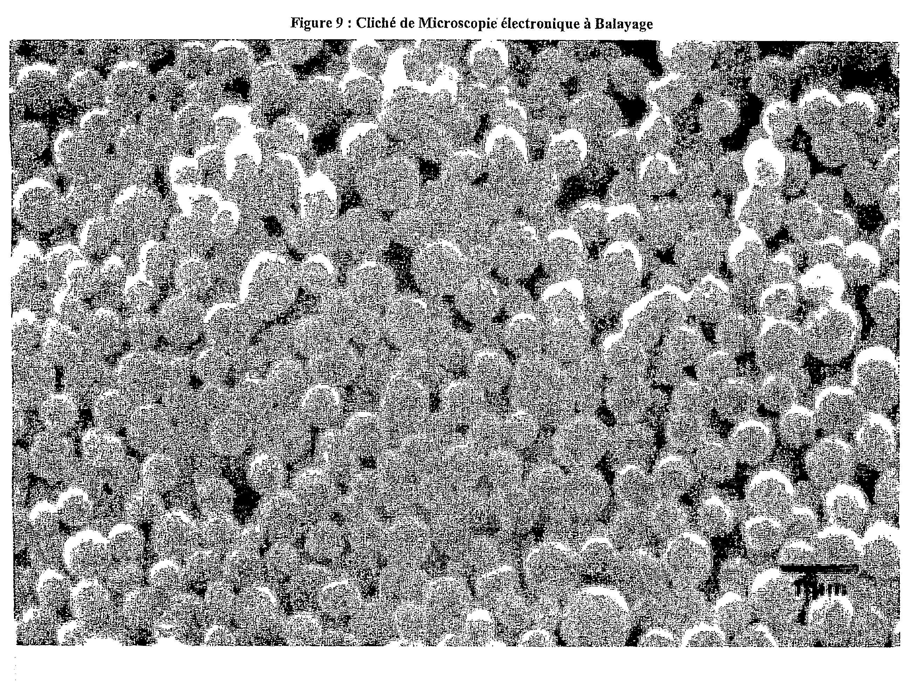

- Figure 9 : Cliché de Microscopie électronique à Balayage de particules sphériques obtenues par copolymérisation des macromonomères A et B en présence des monomères NBH et/ou NBD.

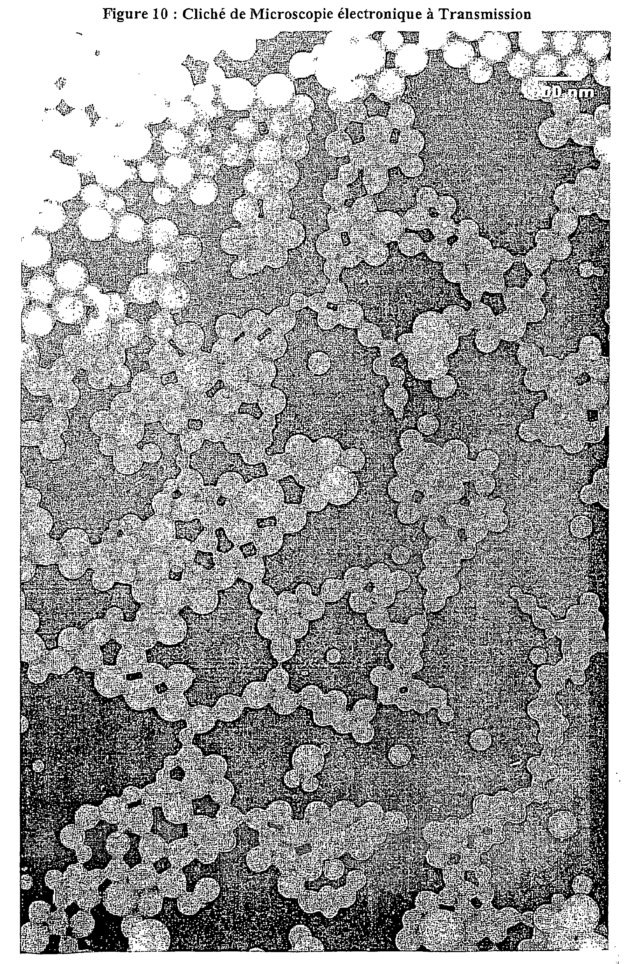

- Figure 10 : Cliché de Microscopie électronique à Transmission de particules sphériques obtenues par copolymérisation des macromonomères A et B en présence des monomères NBH et/ou NBD.

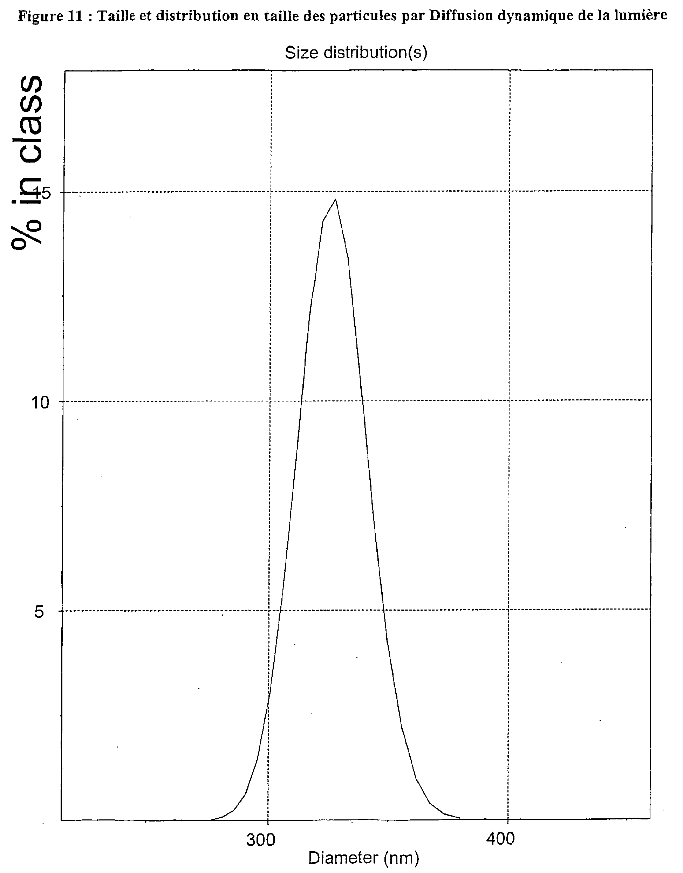

- Figure 11 : Taille et distribution en taille des particules sphériques obtenues par copolymérisation des macromonomères A et B en présence des monomères NBH et/ou NBD, par diffusion dynamique de la lumière.

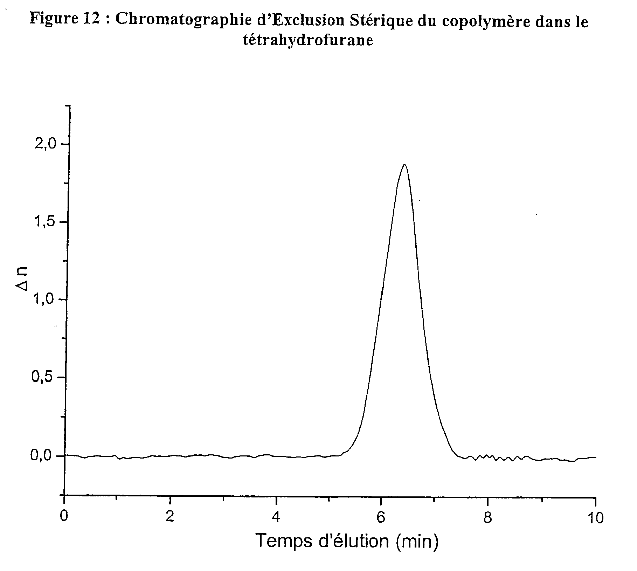

- Figure 12 :Chromatographie d'exclusion stérique des particules sphériques obtenues par copolymérisation des macromonomères A et B en présence des monomères NBH et/ou NBD dans le THF.
- Figure 13 : Représentation d'une particule sphérique selon l'invention dans laquelle le principe actif est emprisonné à l'intérieur de la particule et lié de manière covalente à celle-ci.
- Figure 14 : Illustration de la déstabilisation d'une particule sphérique selon l'invention (ou latex )et relargage du médicament.
- Figure 15 : Observation par Microscopie Electronique à Balayage de l'hydroxyapatite.
- Figure 16: Observation par Microscopie Electronique à Balayage de l'hydroxyapatite + APTES.
- Figure 17: Observation par Microscopie Electronique à Balayage de l'hydroxyapatite + APTES + nanoparticules.
- Figure 18 : Observation par Microscopie Electronique à Balayage de l'hydroxyapatite + APTES + nanoparticules fonctionnalisées après extraction.
- Figure 19: Observation par Microscopie Electronique à Balayage de l'hydroxyapatite + APTES + nanoparticules non fonctionnalisées après extraction.
- Figure 20 (a, b) : spectres XPS desC1 pour les matériaux « A » et « B » respectivement.
   Figure 21 : spectre XPS des N1 pour les surfaces de matériaux B.

## Revendications

1. Biomatériaux comprenant un matériau support à la surface duquel sont liées de façon covalente des particules sphériques d'un diamètre compris entre 10nm et 100µm, lesdites particules étant constituées par des chaînes polymère contenant environ 30 à 10000 unités monomères, identiques ou différentes, dérivées de la polymérisation d'alcènes monocycliques dont le nombre d'atomes de carbone constitutifs du cycle est de 4 à 12, ou d'alcènes polycycliques dont le nombre total d'atomes de carbone constitutifs des cycles est de 6 à 20, lesdites unités monomères étant telles que :
- au moins environ 0,5 % d'entre elles sont substituées par une chaîne R comprenant un polyoxyde d'éthylène de formule (A) le cas échéant lié de façon covalente auxdites unités monomères via un pont hydrolysable
-(CH₂-CH₂-O)ₙ-X (A)
dans laquelle n représente un nombre entier de 50 à 340, notamment de 70 à 200, et X représente une chaîne alkyle ou alkyloxy de 1 à 10 atomes de carbone, comprenant une fonction réactive du type OH, halogène, NH₂, C(O)X₁ dans laquelle X₁ représente un atome d'hydrogène, d'halogène, un groupe OR' ou NHR' dans lequel R' représente un atome d'hydrogène ou une chaîne hydrocarbonée d'environ 1 à 10 atomes de carbone, substituée ou non, ladite fonction réactive étant susceptible de se lier à une fonction réactive située sur le ledit matériau support afin d'assurer la liaison covalente entre ledit matériau et lesdites particules,
- et au moins environ 0,5 % d'entre elles sont substituées par une chaîne R comprenant un polyoxyde d'éthylène de formule (A) susmentionnée dans laquelle ladite fonction réactive est engagée dans une liaison avec un principe actif, ou une molécule biologique telle qu'une protéine, lesdites chaînes R étant liées de façon covalente auxdits monomères.

2. Biomatériaux selon la revendication 1, **caractérisés en ce que** les unités monomères sont dérivées de la polymérisation d'alcènes monocycliques, et sont de formule (Z1) suivante
=[CH-R₁-CH]= (Z1)
dans laquelle R₁ représente une chaîne hydrocarbonée de 2 à 10 atomes de carbone, saturée ou non, lesdites unités monomères étant le cas échéant substituées par une chaîne R, ou directement par un groupe X, tels que définis dans la revendication 1.

3. Biomatériaux selon la revendication 1 ou 2, **caractérisés en ce que** les alcènes monocycliques dont sont issues les unités monomères sont :
- le cyclobutène conduisant à un polymère comprenant des unités monomères de formule (Z1a) suivante : 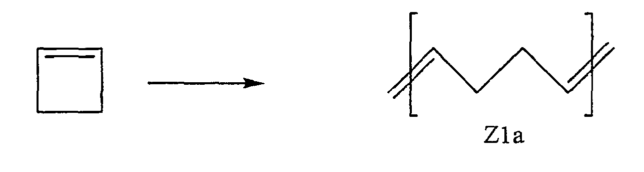
- le cyclopentène conduisant à un polymère comprenant des unités monomères de formule (Z1b) suivante : 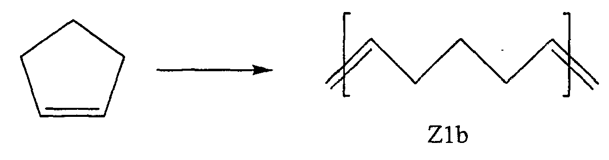
- le cyclopentadiène conduisant à un polymère comprenant des unités monomères de formule (Z1c) suivante 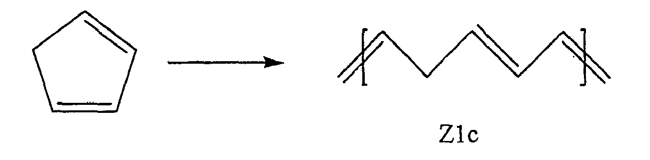
- le cyclohexène conduisant à un polymère comprenant des unités monomères de formule (Z1d) suivante 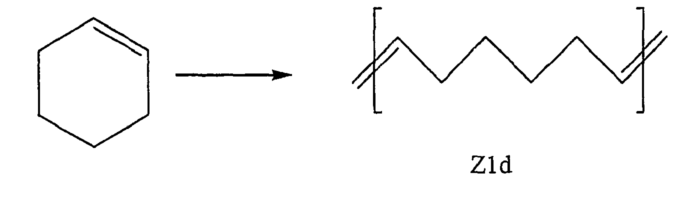
- le cyclohexadiène conduisant à un polymère comprenant des unités monomères de formule (Z1e) suivante 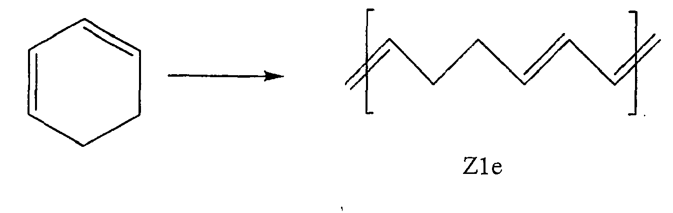
- le cycloheptène conduisant à un polymère comprenant des unités monomères de formule (Z1f) suivante 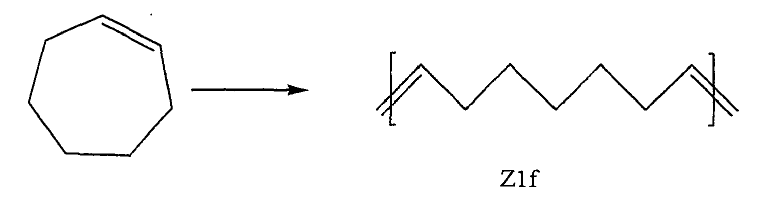
- le cyclooctène conduisant à un polymère comprenant des unités monomères de formule (Z1h) suivante 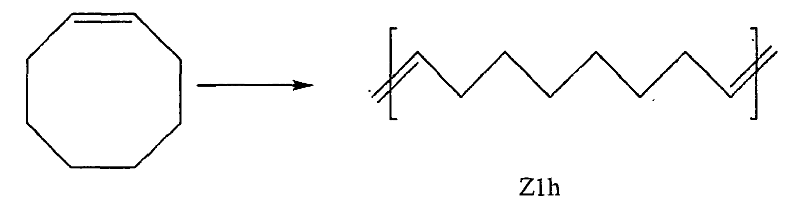
- le cyclooctapolyène, notamment le cycloocta-1,5-diene conduisant à un polymère comprenant des unités monomères de formule (Z1i) suivante 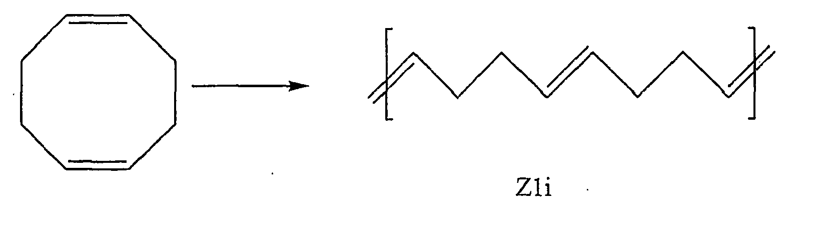
- le cyclononène conduisant à un polymère comprenant des unités monomères de
formule (Z1j) suivante 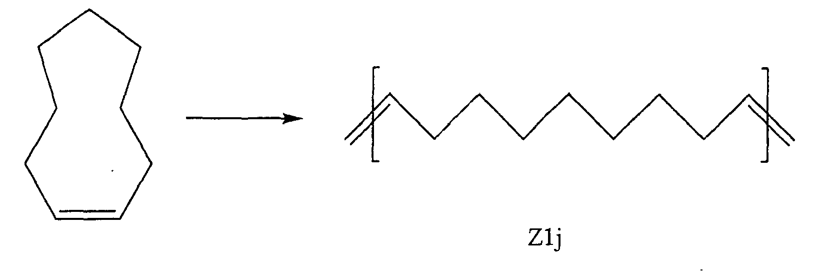
- le cyclononadiène conduisant à un polymère comprenant des unités monomères de formule (Z1k) suivante 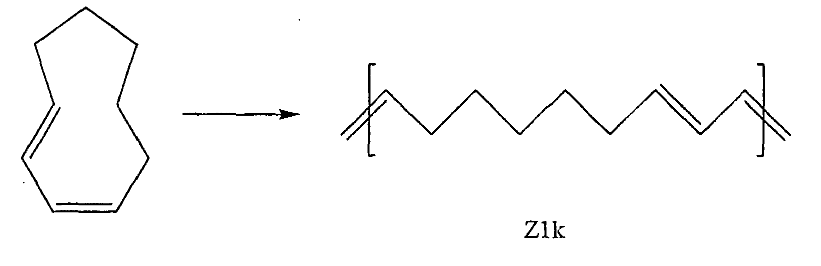
- le cyclodécène conduisant à un polymère comprenant des unités monomères de formule (Z1l) suivante 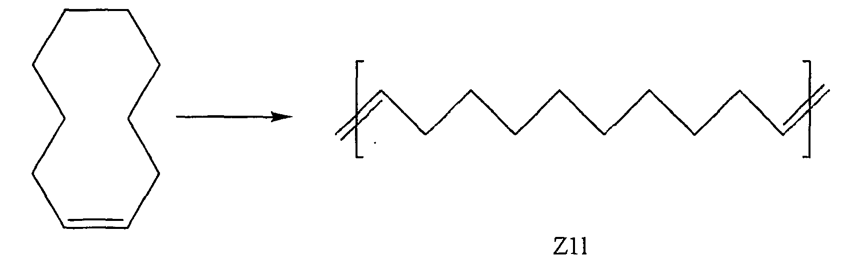
- le cyclodéca-1,5-diène conduisant à un polymère comprenant des unités monomères de formule (Z1m) suivante 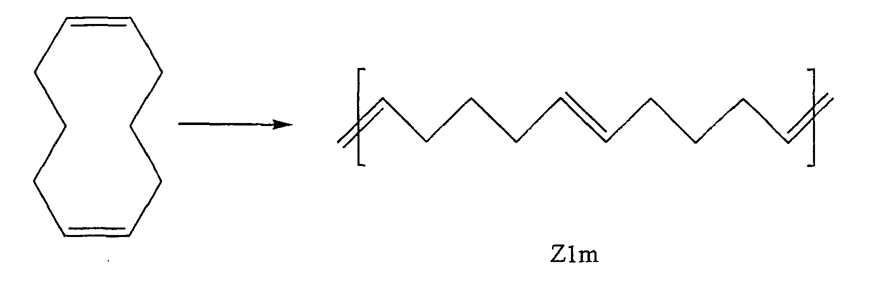
- le cyclododécène conduisant à un polymère comprenant des unités monomères de formule (Z1n) suivante 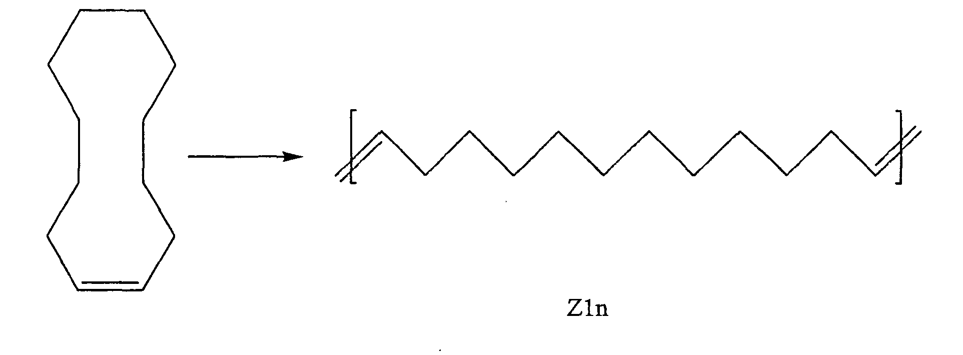
- ou encore l'acétate du 2,3,4,5-tétrahydrooxepin-2-yl, le cyclopentadécène, le paracyclophane, le ferrocenophane.

4. Biomatériaux selon la revendication 1, **caractérisés en ce que** les unités monomères sont dérivées de la polymérisation d'alcènes polycycliques, et sont :
- de formule (Z2) suivante
=[CH-R₂-CH]= (Z2)
dans laquelle R₂ représente:
* un cycle de formule
dans laquelle : 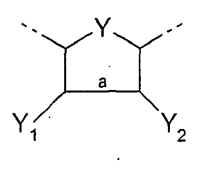
. Y représente -CH₂-, ou un hétéroatome, ou un groupe -CHR-, ou un groupe -CHX-, R et X étant tels que définis dans la revendication 1,
. Y1 et Y2, indépendamment l'un de l'autre, représentent H, ou une chaîne R, ou un groupe X susmentionnés, ou forment en association avec les atomes de carbone qui les portent un cycle de 4 à 8 atomes de carbone, ce cycle étant le cas échéant substitué par une chaîne R, ou un groupe X susmentionnés,
. a représente une simple ou double liaison,
* ou un cycle de formule 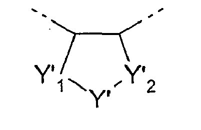 dans laquelle :
. Y' représente -CH₂-, ou un hétéroatome, ou un groupe -CHR-, ou un groupe -CHX-, R et X étant tels que définis ci-dessus,
. Y'1 et Y'2, indépendamment l'un de l'autre, représentent -CH₂-, ou un groupe -C(O), ou un groupe -COR, ou un groupe -C-OX, R et X étant tels que définis ci-dessus,
- de formule (Z3) suivante 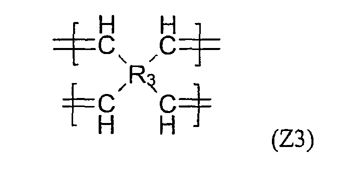 dans laquelle R₃ représente :
* un cycle de formule 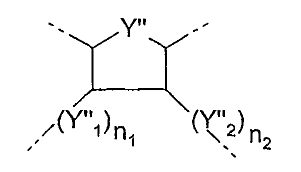 dans laquelle :
. n1 et n2, indépendamment l'un de l'autre, représentent 0 ou 1,
. Y" représente -CH₂-, ou un groupe -CHR-, ou un groupe -CHX-, R et X étant tels que définis ci-dessus,
. Y" 1 et Y"2, indépendamment l'un de l'autre, représentent une chaîne hydrocarbonée de 0 à 10 atomes de carbone,
* ou un cycle de formule 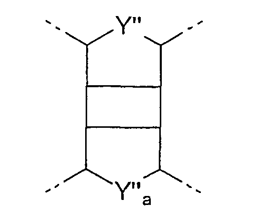 dans laquelle Y" et Y"a, indépendamment l'un de l'autre, représentent -CH₂-, ou un groupe -CHR-, ou un groupe -CHX-, R et X étant tels que définis ci-dessus.
* ou un cycle de formule 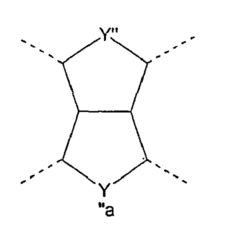 dans laquelle Y" et Y"a, indépendamment l'un de l'autre, représentent -CH₂-, ou un groupe -CHR-, ou un groupe -CHX-, R et X étant tels que définis ci-dessus.

5. Biomatériaux selon la revendication 1 ou 4, **caractérisés en ce que** les alcènes polycycliques dont sont issues les unités monomères sont :
- les monomères contenant un cycle cyclobutène conduisant à un polymère comprenant des unités monomères de formule (Z2a) suivante : 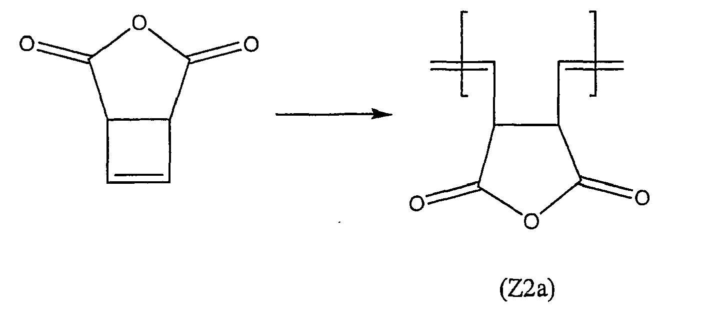
- les monomères contenant un cycle cyclopentène conduisant à un polymère comprenant des unités monomères de formule (Z2b) suivante : 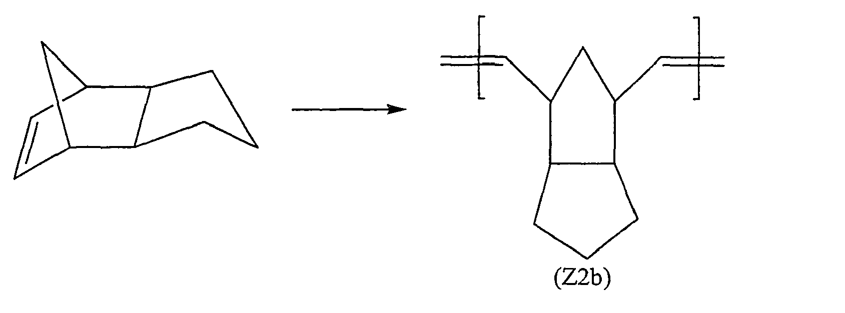
- le norbornène (bicyclo[2.2.1]hept-2-ène) conduisant à un polymère comprenant des unités monomères de formule (Z2c) suivante : 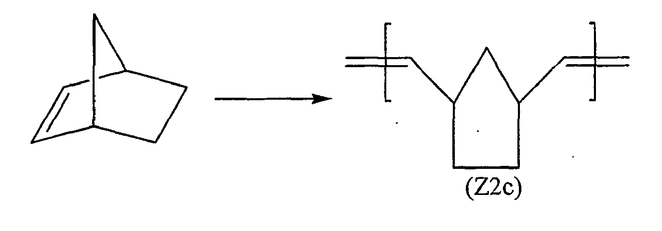
- le norbornadiène conduisant à un polymère comprenant des unités monomères de formule (Z2d) suivante : 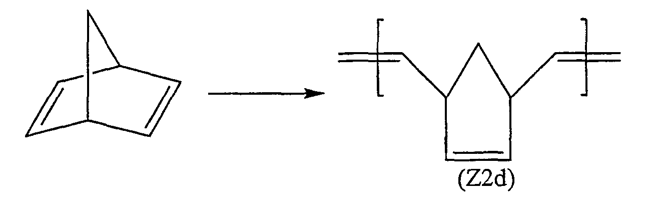
- le 7-oxanorbornène conduisant à un polymère comprenant des unités monomères de formule (Z2e) suivante : 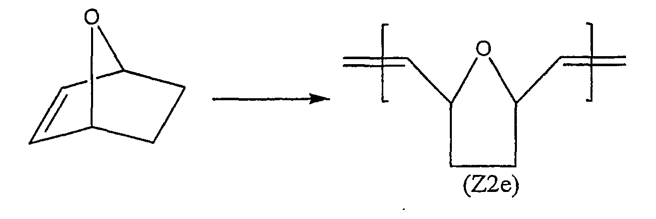
- le 7-oxanorbornadiène conduisant à un polymère comprenant des unités monomères de formule (Z2f) suivante : 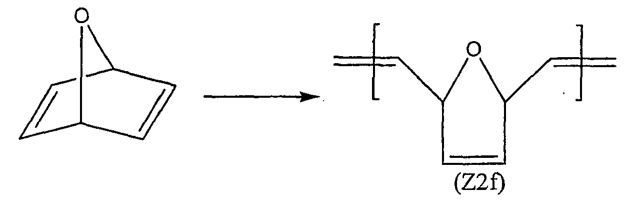
- le dimère du norbornadiène conduisant à un polymère comprenant des unités monomères de formule (Z3 a) suivante : 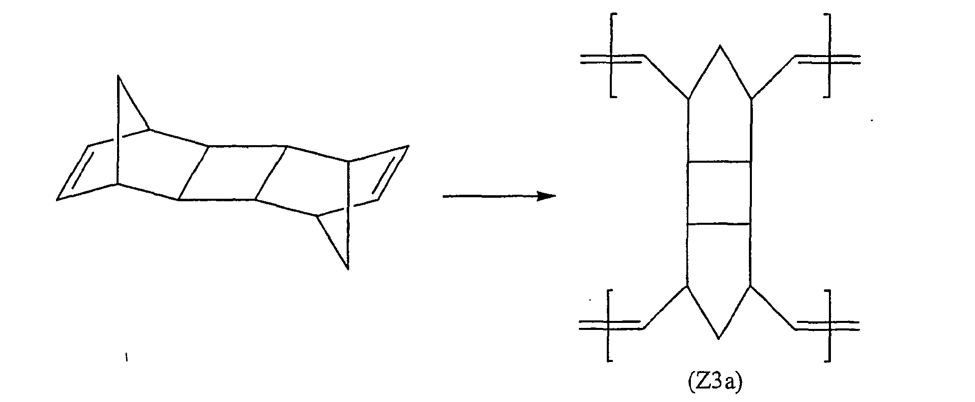
- le dicyclopentadiène conduisant à un polymère comprenant des unités monomères de formule (Z3b) suivante : 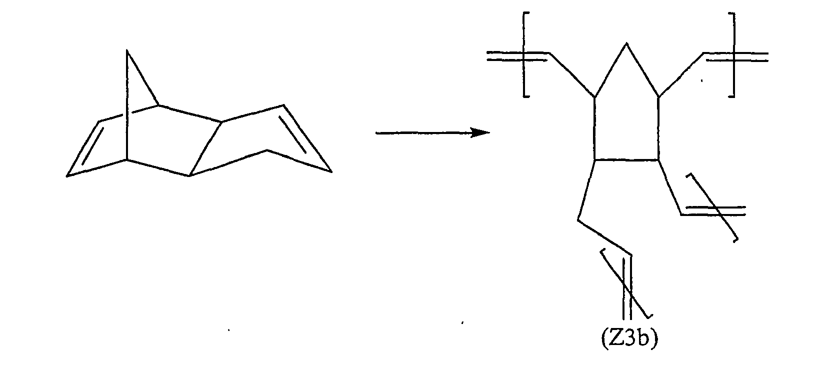
- le tetracyclododécadiene conduisant à un polymère comprenant des unités monomères de formule (Z3c) suivante : 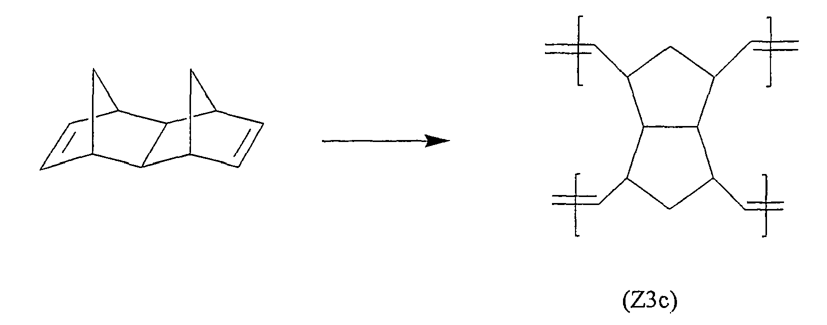
- ou le bicyclo[5.1.0]oct-2-ène, le bicyclo[6.1.0]non-4-ène.

6. Biomatériaux selon l'une des revendications 1 à 5, **caractérisés en ce que** les alcènes mono ou polycycliques dont sont issues les unités monomères sont :
- le norbornène (bicyclo[2.2.1]hept-2-ène) conduisant à un polymère comprenant des unités monomères de formule (Z2c),
- le tetracyclododécadiene conduisant à un polymère comprenant des unités monomères de formule (Z3c),
- le dicyclopentadiène conduisant à un polymère comprenant des unités monomères de formule (Z3b),
- le dimère du norbornadiène conduisant à un polymère comprenant des unités monomères de formule (Z3a),
- le cycloocta-1,5-diene conduisant à un polymère comprenant des unités monomères de formule (Zli).

7. Biomatériaux selon l'une des revendications 1 à 6, **caractérisées en ce que** les particules sphériques comprennent :
- entre 0,5 % jusqu'à 100 % d'unités monomères substituées par une chaîne R telle que définie dans la revendication 1, ladite chaîne R étant identique pour ces monomères, et comprenant une fonction réactive susceptible de se lier à une fonction réactive située sur le ledit matériau support afin d'assurer la liaison covalente entre ledit matériau et lesdites particules,
- et entre 0,5 % et 99,5 % d'unités monomères substituées par une chaîne R telle que définie dans la revendication 1, ladite chaîne R étant identique pour ces monomères, dans laquelle ladite fonction réactive est engagée dans une liaison avec un principe actif, ou une molécule biologique telle qu'une protéine,
- et/ou entre 0,5 % et 99,5 % d'unités monomères substituées directement par un groupe X tel que défini dans la revendication 1, ce groupe X de ces monomères étant identique ou différent du groupe X de la chaîne R des monomères précédents,
- et/ou entre 1 % et 99,5 % d'unités monomères non substituées,
le total des pourcentages des différents monomères susmentionnés faisant 100 %.

8. Biomatériaux selon l'une des revendications 1 à 7, **caractérisés en ce que** les chaînes R substituant les monomères sont représentées par la formule
-CH₂-O-(CH₂-CH₂-O)ₙ- CH₂-CH₂-O-X
dans laquelle n est tel que défini dans la revendication 1, et X représente H, -CH₂-COOH, -CH₂-COCl, ou -CH₂-COY, Y représentant un principe actif, ou une molécule biologique telle qu'une protéine.

9. Biomatériaux selon l'une des revendications 1 à 8, **caractérisées en ce que** la ou les chaînes R comprennent un polyoxyde d'éthylène de formule (A) lié de façon covalente à un pont hydrolysable choisi parmi les enchaînements de 1 à 10 motifs d'ε-caprolactone, ou de fonctions -OC(O)-, -C(O)OC(O)-, -C(O)-NH-.

10. Biomatériaux selon l'une des revendications 1 à 9, **caractérisés en ce que** la ou les chaînes R comprenant un polyoxyde d'éthylène de formule (A) lié de façon covalente à un pont hydrolysable choisi parmi les enchaînements de 1 à 10 motifs d'ε-caprolactone, sont représentées par la formule
-CH₂-(O-CO-(CH₂)₅)ₜ-O-CO-(CH₂)₅-O-CO-(CH₂)₂-CO-O-(CH₂-CH₂-O)ₙ-(CH₂)₂-O-X
dans laquelle t représente un nombre entier compris entre 1 et 10, et X représente H, -CH₂-COOH, -CH₂-COCl, ou -CH₂-COY, Y représentant un principe actif, ou une molécule biologique telle qu'une protéine.

11. Biomatériaux selon l'une des revendications 1 à 10, **caractérisés en ce que** le matériau support est choisi parmi :
- les métaux, tels que le Titane,
- les alliages métalliques, notamment les alliages à mémoire de forme ou non tels que les alliages Ni-Ti
- les polymères, tels que le polyéthylène téréphtalate (PET), polytétrafluoroéthylène (PTFE), polyfuorure de vinylidène (PVDF), polyéther éthercétone (PEEK),
- les copolymères, tels que le copolymère éthylène-acétate de vinyle (EVA), le copolymère fluorure de vinylidène- hexafluoropropylène P(VDF-HFP), le poly(acide lactique)-co-poly(acide glycolique) (PLA-PGA)
- les céramiques, tels que les hydroxyapatites, ou composées d'hydroxyapatites et de phosphate tricalcique dans des proportions variées, notamment dans les proportions 50/50.

12. Biomatériaux selon l'une des revendications 1 à 11, **caractérisés en ce que** la fonction réactive située sur le matériau support afin d'assurer la liaison covalente entre ledit matériau et lesdites particules en réagissant avec la fonction réactive de ces dernières, est du type OH, halogène, NH₂, C(O)X'₁ dans laquelle X'₁ représente un atome d'hydrogène, d'halogène, un groupe OR" ou NHR" dans lequel R" représente un atome d'hydrogène ou une chaîne hydrocarbonée de 1 à 10 atomes de carbone, substituée ou non, pour former une liaison de type -O-C(O)-, -NH-C(O)-, -C(O)-NH-, -C(O)O-, ou -C(OC)₂-, avec la fonction réactive desdites particules.

13. Biomatériaux selon l'une des revendications 1 à 12, **caractérisés en ce que** la fonction réactive du matériau support est située sur une chaîne alkyle de 1 à 10 atomes de carbone, greffée sur ledit matériau, substituée ou non, et comprenant le cas échéant un ou plusieurs hétéroatomes, notamment O, et Si, dans ladite chaîne.

14. Biomatériaux selon l'une des revendications 1 à 13, **caractérisés en ce que** :
- la fonction réactive du matériau est une fonction NH₂ située sur une molécule d'aminopropyltriéthoxysilane greffée sur le matériau conformément aux formules suivantes : 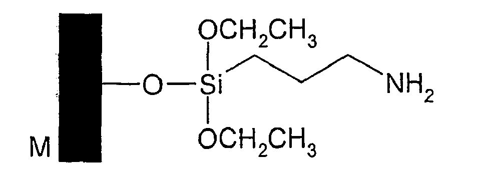 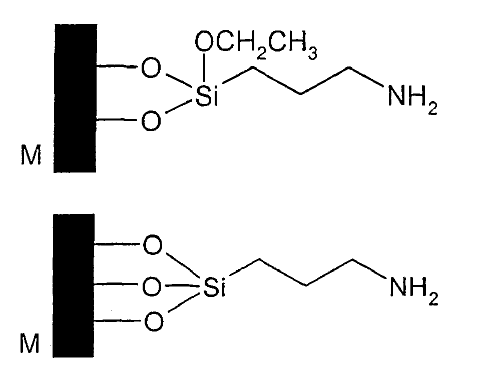 dans lesquelles M représente un oxyde métallique, ou une céramique telle que l'hydroxyapatite, ou tout autre polymère comportant des sites OH en leur surface (par nature ou par le biais d'une préfonctionnalisation),
- la fonction réactive du matériau est une fonction NH₂ située sur une surface préfonctionnalisée par de l'acide acrylique laquelle est couplée à un bras espaceur bifonctionnel tel que le bNH₂PEG (O, O'-bis-(2-aminopropyl)-polyéthylène glycol 500.

15. Biomatériaux selon l'une des revendications 1 à 14, **caractérisés en ce que** le principe actif est choisi parmi les molécules utilisées en thérapeutique, cosmétique, parfumerie, ou pour les revêtements de surface, tels que les peintures et les antifoulings.

16. Biomatériaux selon l'une des revendications 1 à 15, **caractérisés en ce que** le principe actif est choisi parmi les molécules utilisées en thérapeutique, notamment parmi ceux des classes thérapeutiques suivantes : antibiotique, anti-inflammatoire, antimitotique, hormones, facteurs de croissance.

17. Utilisation de biomatériaux selon l'une des revendications 1 à 16, pour la préparation de dispositifs médicaux implantables, notamment sous forme d'implants, de prothèses, de stents, ou de ciments, notamment en chirurgie vasculaire, endovasculaire, ou osseuse.

18. Implants, prothèses, stents vasculaires, ou ciments, comprenant des biomatériaux selon l'une des revendications 1 à 16.

## Claims

1. Biomaterials comprising a support material to the surface of which there are covalently bonded spherical particles of a diameter between 10 nm and 100 µm inclusive, said particles being constituted by polymer chains containing about 30 to 10000 monomer units, which are the same or different, derived from the polymerisation of monocyclic alkenes, the number of carbon atoms constituting the ring of which is from 4 to 12, or of polycyclic alkenes, the total number of carbon atoms constituting the rings of which is from 6 to 20, said monomer units being such that:
- at least about 0.5 % of them are substituted by a chain R comprising a poly(ethylene oxide) of formula (A), optionally covalently bonded to said monomer units via a hydrolysable bridge
-(CH₂-CH₂-O)ₙ-X (A)
wherein n represents an integer from 50 to 340, especially from 70 to 200, and X represents an alkyl or alkyloxy chain of 1 to 10 carbon atoms, having a reactive function of the type OH, halogen, NH₂, C(O)X₁ wherein X₁ represents a hydrogen atom, a halogen atom, a group OR' or NHR' wherein R' represents a hydrogen atom or a substituted or unsubstituted hydrocarbon chain of about 1 to 10 carbon atoms, said reactive function being capable of bonding to a reactive function located on said support material in order to establish the covalent bonding between said material and said particles,
- and at least about 0.5 % of them are substituted by a chain R comprising a poly(ethylene oxide) of the above-mentioned formula (A) wherein said reactive function is engaged in a bond with an active ingredient or a biological molecule such as a protein, said chains R being covalently bonded to said monomers.

2. Biomaterials according to claim 1, **characterised in that** the monomer units are derived from the polymerisation of monocyclic alkenes and are of the following formula (Z1)
=[CH-R₁-CH]= (Z1)
wherein R₁ represents a saturated or unsaturated hydrocarbon chain of 2 to 10 carbon atoms, said monomer units optionally being substituted by a chain R, or directly by a group X, as are defined in claim 1.

3. Biomaterials according to claim 1 or 2, **characterised in that** the monocyclic alkenes from which the monomer units originate are:
- cyclobutene resulting in a polymer comprising monomer units of the following formula (Z1a): 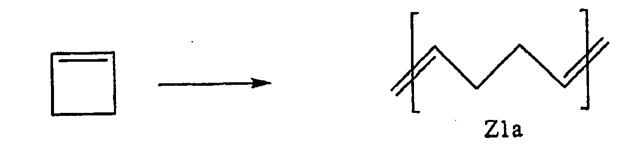
- cyclopentene resulting in a polymer comprising monomer units of the following formula (Z1b): 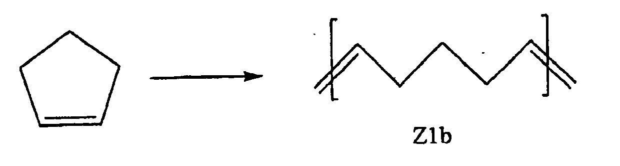
- cyclopentadiene resulting in a polymer comprising monomer units of the following formula (Z1c) 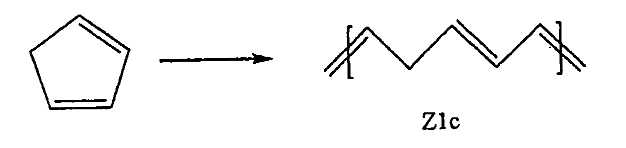
- cyclohexene resulting in a polymer comprising monomer units of the following formula (Z1d) 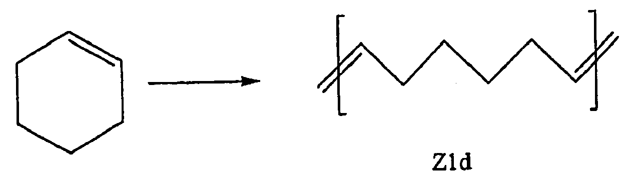
- cyclohexadiene resulting in a polymer comprising monomer units of the following formula (Z1e) 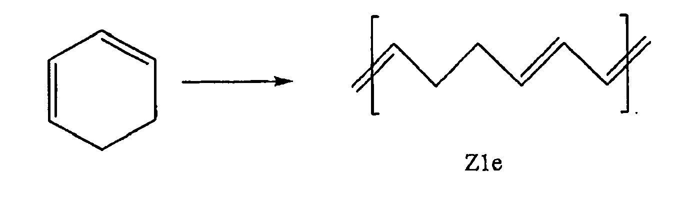
- cycloheptene resulting in a polymer comprising monomer units of the following formula (Z1f) 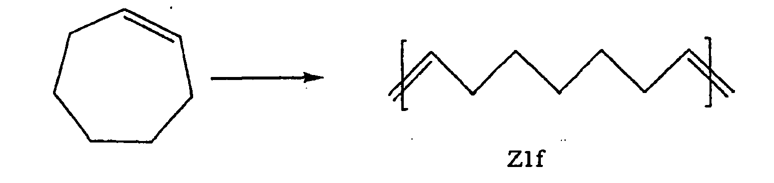
- cyclooctene resulting in a polymer comprising monomer units of the following formula (Z1h) 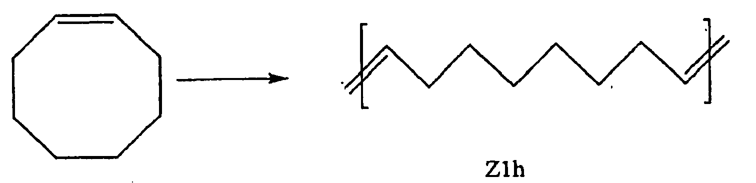
- cyclooctapolyene, especially cycloocta-1,5-diene, resulting in a polymer comprising monomer units of the following formula (Z1i) 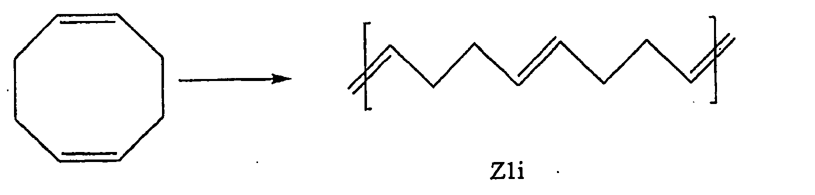
- cyclononene resulting in a polymer comprising monomer units of the following formula (Z1j) 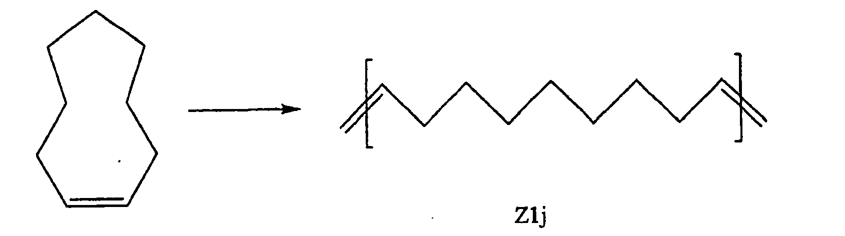
- cyclononadiene resulting in a polymer comprising monomer units of the following formula (Z1k) 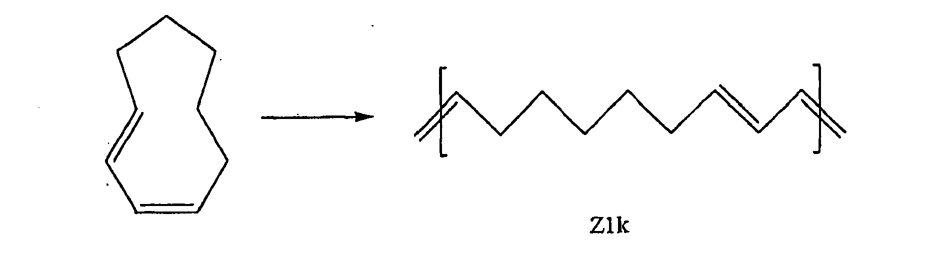
- cyclodecene resulting in a polymer comprising monomer units of the following formula (Z1l) 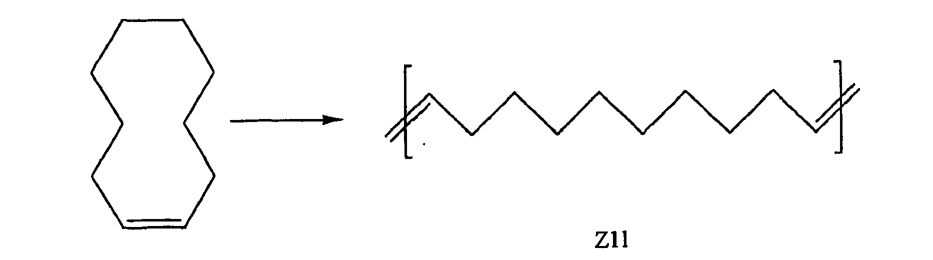
- cyclodeca-1,5-diene resulting in a polymer comprising monomer units of the following formula (Z1m) 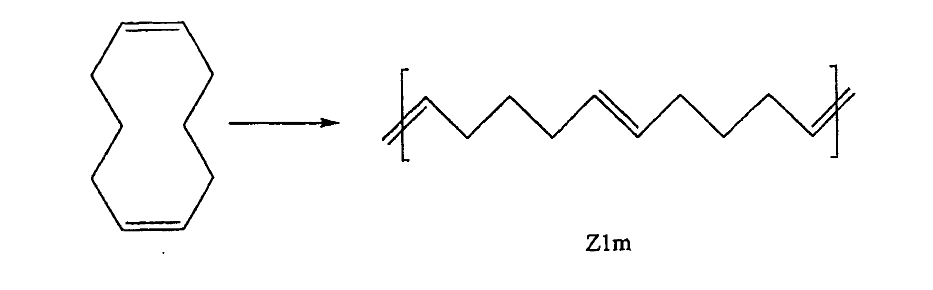
- cyclododecene resulting in a polymer comprising monomer units of the following formula (Z1n) 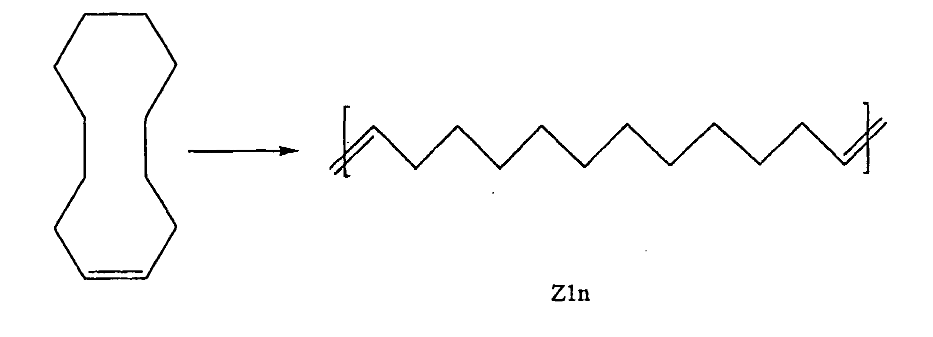
- or also 2,3,4,5-tetrahydrooxepin-2-yl acetate, cyclopentadecene, paracyclophane, ferrocenophane.

4. Biomaterials according to claim 1, **characterised in that** the monomer units are derived from the polymerisation of polycyclic alkenes and are:
- of the following formula (Z2)
=[CH-R₂-CH]= (Z2)
wherein R₂ represents:
* a ring of formula 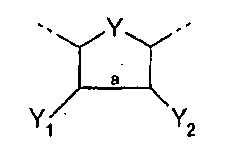 wherein:
. Y represents -CH₂-, or a hetero atom, or a group -CHR-, or a group -CHX-, R and X being as defined in claim 1,
. Y1 and Y2, each independently of the other, represent H or a chain R or a group X as are mentioned above or, in association with the carbon atoms carrying them, form a ring of 4 to 8 carbon atoms, this ring optionally being substituted by a chain R or a group X as are mentioned above,
a represents a single or double bond,
* or a ring of formula 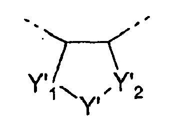 wherein:
.Y' represents -CH₂-, or a hetero atom, or a group -CHR-, or a group -CHX-, R and X being as defined hereinbefore,
. Y'1 and Y'2, each independently of the other, represent --CH2-, or a group -C(O), or a group -COR, or a group -C-OX, R and X being as defined hereinbefore,
- of the following formula (Z3) 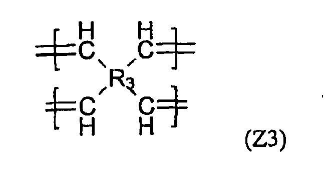 wherein R₃ represents:
* a ring of formula 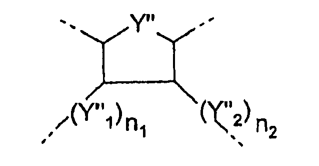 wherein:
. n1 and n2, each independently of the other, represent 0 or 1,
. Y" represents -CH₂-, or a group -CHR-, or a group -CHX-, R and X being as defined hereinbefore,
. Y"1 and Y"2, each independently of the other, represent a hydrocarbon chain of 0 to 10 carbon atoms,
* or a ring system of formula 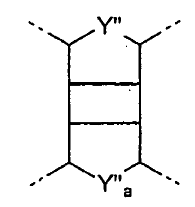 wherein Y" and Y"a, each independently of the other, represent -CH₂-, or a group -CHR-, or a group -CHX-, R and X being as defined hereinbefore,
* or a ring system of formula 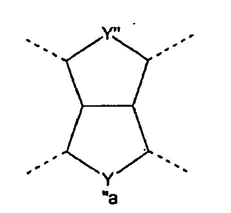 wherein Y" and Y"a, each independently of the other, represent -CH₂-, or a group -CHR-, or a group -CHX-, R and X being as defined hereinbefore.

5. Biomaterials according to claim 1 or 4, **characterised in that** the polycyclic alkenes from which the monomer units originate are:
- monomers containing a cyclobutene ring resulting in a polymer comprising monomer units of the following formula (Z2a): 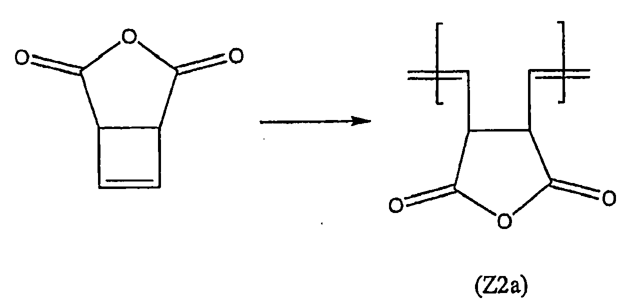
- monomers containing a cyclopentene ring resulting in a polymer comprising monomer units of the following formula (Z2b): 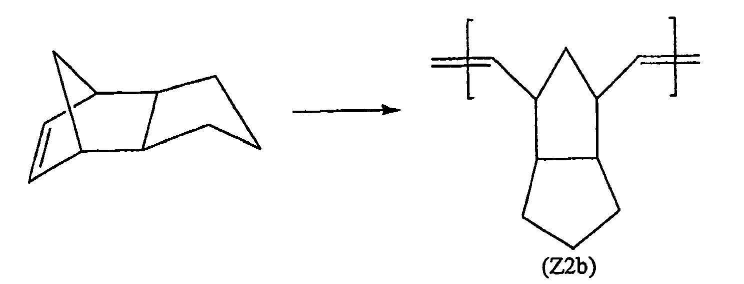
- norbornene (bicyclo[2.2.1]hept-2-ene) resulting in a polymer comprising monomer units of the following formula (Z2c): 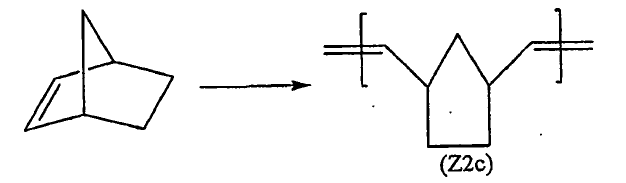
- norbornadiene resulting in a polymer comprising monomer units of the following formula (Z2d): 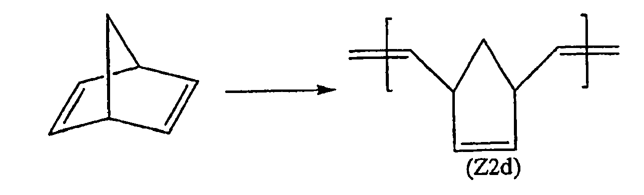
- 7-oxanorbornene resulting in a polymer comprising monomer units of the following formula (Z2e): 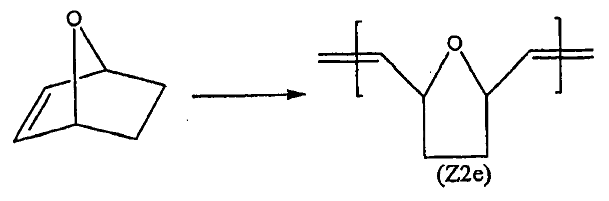
- 7-oxanorbornadiene resulting in a polymer comprising monomer units of the following formula (Z2f): 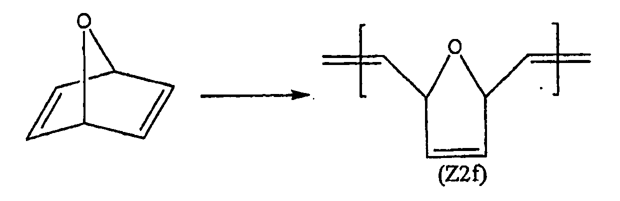
- norbornadiene dimer resulting in a polymer comprising monomer units of the following formula (Z3a): 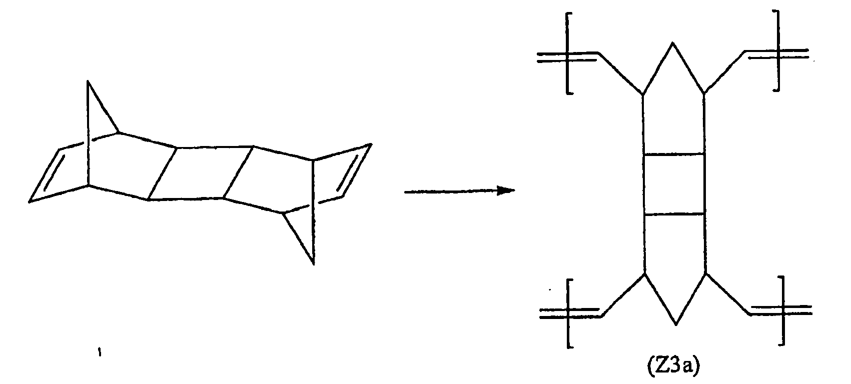
- dicyclopentadiene resulting in a polymer comprising monomer units of the following formula (Z3b): 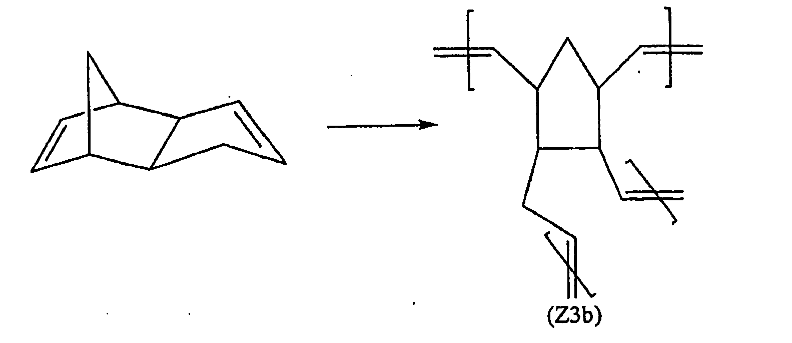
- tetracyclododecadiene resulting in a polymer comprising monomer units of the following formula (Z3c): 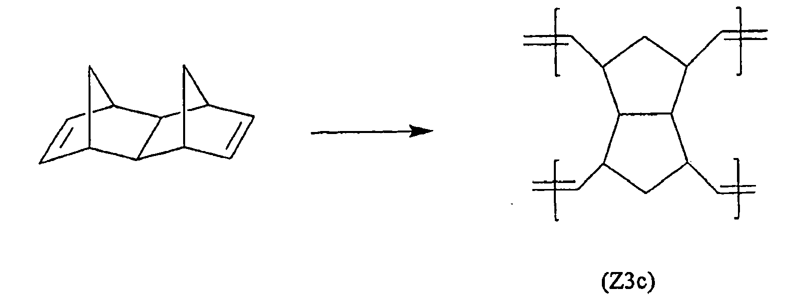
- or bicyclo[5.1.0]oct-2-ene, bicyclo[6.1.0]non-4-ene.

6. Biomaterials according to one of claims 1 to 5, **characterised in that** the mono- or poly-cyclic alkenes from which the monomer units originate are:
- norbornene (bicyclo[2.2.1]hept-2-ene) resulting in a polymer comprising monomer units of formula (Z2c),
- tetracyclododecadiene resulting in a polymer comprising monomer units of formula (Z3c),
- dicyclopentadiene resulting in a polymer comprising monomer units of formula (Z3b),
- norbornadiene dimer resulting in a polymer comprising monomer units of formula (Z3a),
- cycloocta-1,5-diene resulting in a polymer comprising monomer units of formula (Z1i).

7. Biomaterials according to one of claims 1 to 6, **characterised in that** the spherical particles comprise:
- from 0.5 % up to 100 % monomer units substituted by a chain R as defined in claim 1, said chain R being the same for these monomers and having a reactive function capable of bonding to a reactive function located on said support material in order to establish covalent bonding between said material and said particles,
- and between 0.5 % and 99.5 % monomer units substituted by a chain R as defined in claim 1, said chain R being the same for these monomers, wherein said reactive function is engaged in a bond with an active ingredient or a biological molecule such as a protein,
- and/or between 0.5 % and 99.5 % monomer units substituted directly by a group X as defined in claim 1, that group X of these monomers being the same as or different to the group X of the chain R of the above monomers,
- and/or between 1 % and 99.5 % unsubstituted monomer units,
the sum of the percentages of the various above-mentioned monomers being 100 %.

8. Biomaterials according to one of claims 1 to 7, **characterised in that** the chains R substituting the monomers are represented by the formula
-CH₂-O-(CH₂-CH₂-O)ₙ-CH₂-CH₂-O-X
wherein n is as defined in claim 1, and X represents H, -CH₂-COOH, -CH₂-COCl or -CH₂-COY, Y representing an active ingredient or a biological molecule such as a protein.

9. Biomaterials according to one of claims 1 to 8, **characterised in that** the chain or chains R comprise(s) a poly(ethylene oxide) of formula (A) covalently bonded to a hydrolysable bridge selected from concatenations of 1 to 10 moieties of ε-caprolactone, or of functions -OC(O)-, -C(O)OC(O)-, -C(O)-NH-.

10. Biomaterials according to one of claims 1 to 9, **characterised in that** the chain or chains R comprising a poly(ethylene oxide) of formula (A) covalently bonded to a hydrolysable bridge selected from concatenations of 1 to 10 moieties of ε-caprolactone are represented by the formula
-CH₂-(O-CO-(CH₂)₅)ₜ-O-CO-(CH₂)₅-O-CO-(CH₂)₂-CO-O-(CH₂-CH₂-O)ₙ-(CH₂)₂-O-X
wherein t represents an integer between 1 and 10 inclusive, and X represents H, -CH₂-COOH, -CH₂-COCI or -CH₂-COY, Y representing an active ingredient or a biological molecule such as a protein.

11. Biomaterials according to one of claims 1 to 10, **characterised in that** the support material is selected from:
- metals, such as titanium,
- metal alloys, especially alloys having shape memory or not, such as Ni-Ti alloys
- polymers, such as polyethylene terephthalate (PET), polytetrafluoroethylene (PTFE), polyvinylidene fluoride (PVDF), polyether ether ketone (PEEK),
- copolymers, such as ethylene-vinyl acetate copolymer (EVA), vinylidene fluoride-hexafluoropropylene copolymer P(VDF-HFP), poly(lactic acid)-copoly(glycolic acid) (PLA-PGA)
- ceramics, such as hydroxyapatites, or those composed of hydroxyapatites and tricalcium phosphate in various proportions, especially in proportions of 50/50.

12. Biomaterials according to one of claims 1 to 11, **characterised in that** the reactive function located on the support material in order to establish the covalent bonding between said material and said particles by reacting with the reactive function of the latter is of the type OH, halogen, NH₂, C(O)X'₁ wherein X'₁ represents a hydrogen atom, a halogen atom, a group OR" or NHR" wherein R" represents a hydrogen atom or a substituted or unsubstituted hydrocarbon chain of 1 to 10 carbon atoms to form a linkage of the type -O-C(O)-, -NH-C(O)-, -C(O)-NH-, -C(O)O- or -C(OC)₂- with the reactive function of said particles.

13. Biomaterials according to one of claims 1 to 12, **characterised in that** the reactive function of the support material is located on a substituted or unsubstituted alkyl chain of 1 to 10 carbon atoms grafted onto said material and optionally containing one or more hetero atoms, especially O and Si, in said chain.

14. Biomaterials according to one of claims 1 to 13, **characterised in that**:
- the reactive function of the material is an NH₂ function located on a molecule of aminopropyltriethoxysilane grafted onto the material in conformity with the following formulae: 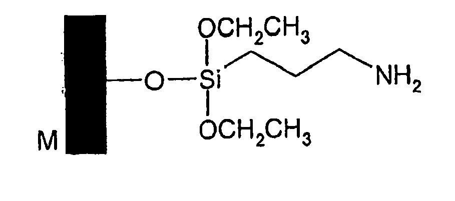 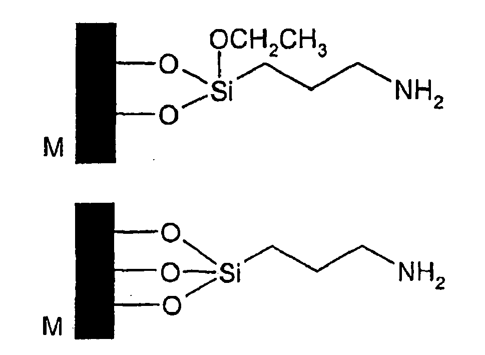 wherein M represents a metal oxide or a ceramic such as hydroxyapatite or any other polymer having OH sites on its surface (naturally or as a result of prefunctionalisation),
- the reactive function of the material is an NH₂ function located on a surface prefunctionalised by acrylic acid and coupled to a bifunctional spacer arm such as bNH₂PEG (O,O'-bis-(2-aminopropyl)-polyethylene glycol 500).

15. Biomaterials according to one of claims 1 to 14, **characterised in that** the active ingredient is selected from molecules used in therapeutics, cosmetics, perfumery or for surface coatings such as paints and antifoulings.

16. Biomaterials according to one of claims 1 to 15, **characterised in that** the active ingredient is selected from molecules used in therapeutics, especially from those of the following therapeutic classes: antibiotic, anti-inflammatory, antimitotic, hormones, growth factors.

17. Use of biomaterials according to one of claims 1 to 16 in the production of implantable medical devices, especially in the form of implants, prostheses, stents or cements, especially in vascular, endovascular or bone surgery.

18. Implants, prostheses, vascular stents or cements, comprising biomaterials according to one of claims 1 to 16.

## Patentansprüche

1. Biomaterial, das ein Trägermaterial umfasst, an dessen Oberfläche kugelförmige Teilchen mit einem Durchmesser von 10 nm bis 100 µm kovalent gebunden sind, die aus Polymerketten gebildet sind, die etwa 30 bis 10 000 gegebenenfalls voneinander verschiedene Monomereinheiten enthalten, die sich aus der Polymerisation monocyclischer Alkene, deren Anzahl an den Cyclus aufbauenden Kohlenstoffatomen 4 bis 12 beträgt, oder polycyclischer Alkene, deren Gesamtzahl an die Cyclen aufbauenden Kohlenstoffatomen 6 bis 20 beträgt, ableiten, wobei diese Monomereinheiten derart sind, dass:
- mindestens etwa 0,5 % davon mit einer Kette R substituiert sind, die ein Polyethylenoxid umfasst, das gegebenenfalls kovalent mit den Monomereinheiten über eine hydrolysierbare Brücke verbunden ist und die Formel (A)
-(CH₂-CH₂-O)ₙ-X (A)
besitzt, in welcher n eine ganze Zahl von 50 bis 340, insbesondere 70 bis 200, und X eine Alkyl- oder Alkyloxykette mit 1 bis 10 Kohlenstoffatomen bedeutet, die eine reaktive Funktion vom Typ OH, Halogen, NH₂ und C(O)X₁ umfasst, in welcher X₁ ein Wasserstoffatom, Halogenatom und eine OR'- oder NHR'-Gruppe bedeutet, in welcher R' ein Wasserstoffatom oder eine gegebenenfalls substituierte Kohlenwasserstoffkette mit etwa 1 bis 10 Kohlenstoffatomen bedeutet, wobei die reaktive Funktion in der Lage ist, sich mit einer reaktiven Funktion, die sich auf dem Trägermaterial befindet, zu verbinden, um die kovalente Bindung dieses Materials mit den Teilchen sicherzustellen, und
- mindestens etwa 0,5 % davon mit einer Kette R substituiert sind, die ein Polyethylenoxid mit obiger Formel (A) umfasst, und in welcher die reaktive Funktion eine Bindung mit einem Wirkstoff oder einem biologischen Molekül wie einem Protein eingegangen ist, wobei die Ketten R mit den Monomeren kovalent verbunden sind.

2. Biomaterial nach Anspruch 1, **dadurch gekennzeichnet, dass** die Monomereinheiten sich aus der Polymerisation monocyclischer Alkene ableiten und die Formel (Z1)
=[CH-R₁-CH]= (Z1)
besitzen, in welcher R₁ eine gegebenenfalls ungesättigte Kohlenwasserstoffkette mit 2 bis 10 Kohlenstoffatomen bedeutet, wobei diese Monomereinheiten gegebenenfalls mit einer Kette R oder direkt mit einer Gruppe X substituiert sind, wie sie in Anspruch 1 definiert ist.

3. Biomaterial nach Anspruch 1 oder 2, **dadurch gekennzeichnet, dass** die monocyclischen Alkene, aus denen die Monomereinheiten hervorgegangen sind:
- Cyclobuten, das ein Polymer ergibt, das Monomereinheiten mit der Formel (Z1a) 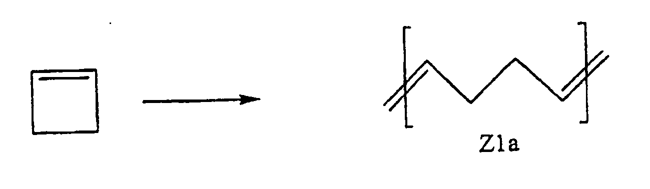 umfasst,
- Cyclopenten, das ein Polymer ergibt, das Monomereinheiten mit der Formel (Z1b) 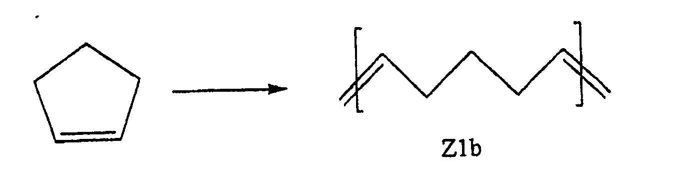 umfasst,
- Cyclopentadien, das ein Polymer ergibt, das Monomereinheiten mit der Formel (Z1c) 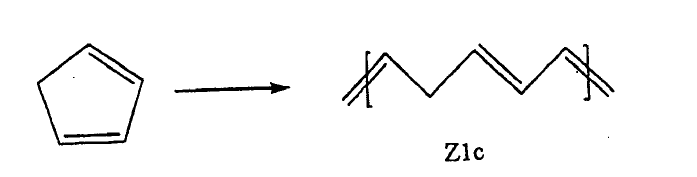 umfasst,
- Cyclohexen, das ein Polymer ergibt, das Monomereinheiten mit der Formel (Z1d) 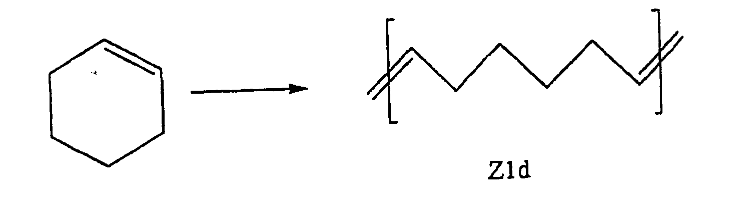 umfasst,
- Cyclohexadien, das ein Polymer ergibt, das Monomereinheiten mit der Formel (Z1e) 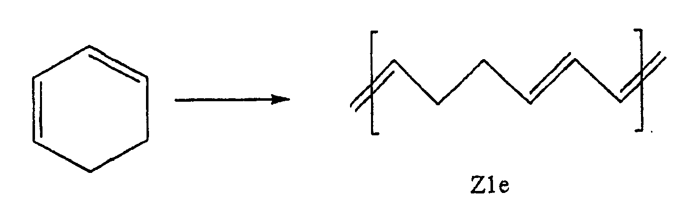 umfasst,
- Cyclohepten, das ein Polymer ergibt, das Monomereinheiten mit der Formel (Z1f) 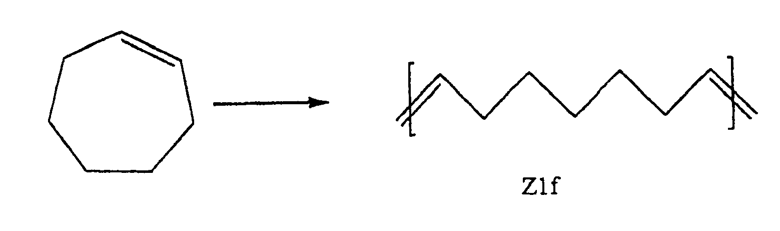 umfasst,
- Cycloocten, das ein Polymer ergibt, das Monomereinheiten mit der Formel (Z1h) 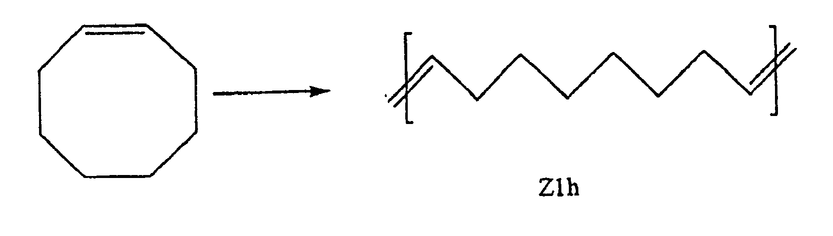 umfasst,
- Cyclooctapolyen, insbesondere Cycloocta-1,5-dien, das ein Polymer ergibt, das Monomereinheiten mit der Formel (Z1i) 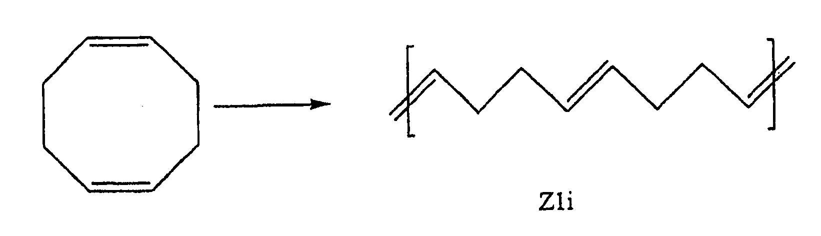 umfasst,
- Cyclononen, das ein Polymer ergibt, das Monomereinheiten mit der Formel (Z1j) 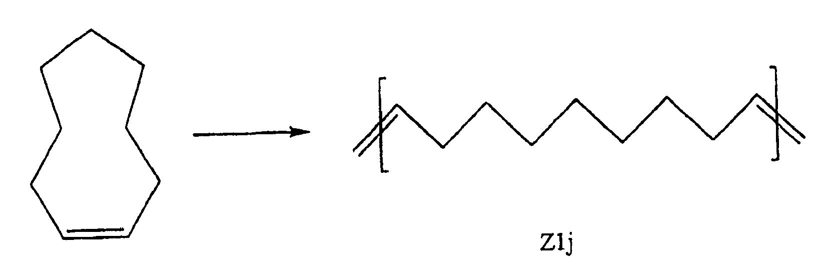 umfasst,
- Cyclononadien, das ein Polymer ergibt, das Monomereinheiten mit der Formel (Z1k) 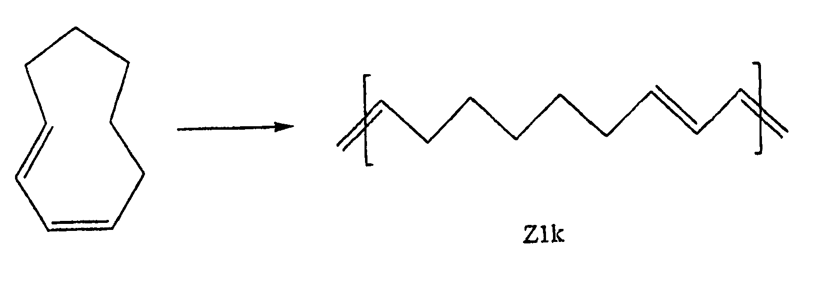 umfasst,
- Cyclodecen, das ein Polymer ergibt, das Monomereinheiten mit der Formel (Z1l) 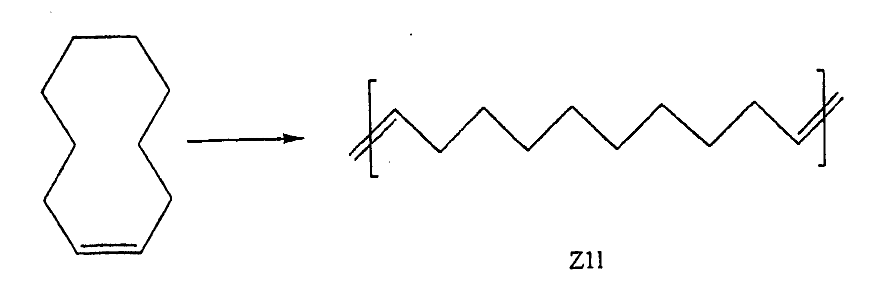 umfasst,
- Cyclodeca-1,5-dien, das ein Polymer ergibt, das Monomereinheiten mit der Formel (Z1m) 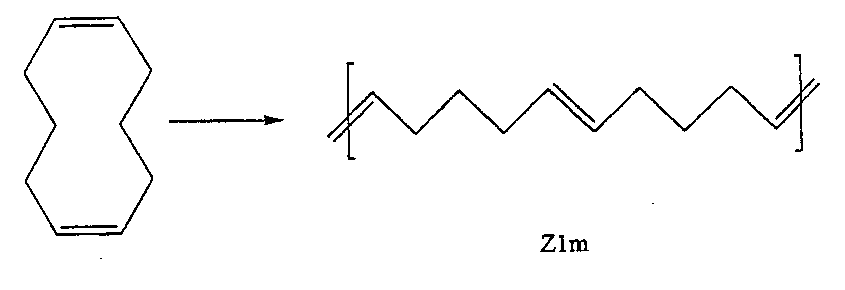 umfasst,
- Cyclododecen, das ein Polymer ergibt, das Monomereinheiten mit der Formel (Z1n) 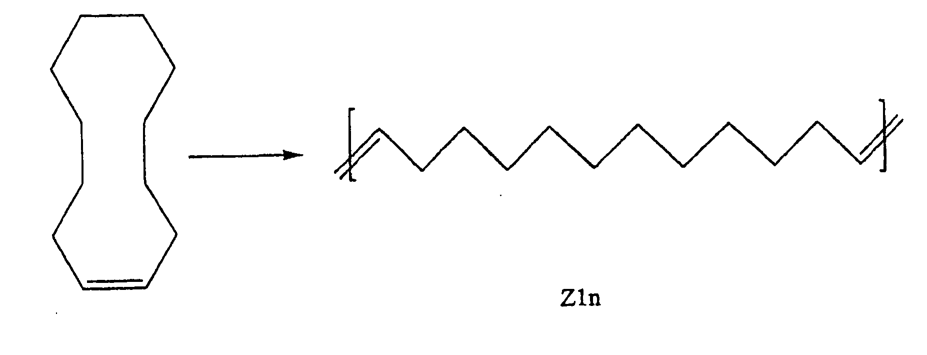 umfasst,
oder 2,3,4,5-Tetrahydrooxepin-2-ylacetat, Cyclopentadecen, Paracyclophan und Ferrocenophan sind.

4. Biomaterial nach Anspruch 1, **dadurch gekennzeichnet, dass** die Monomereinheiten sich aus der Polymerisation polycyclischer Alkene ableiten und.
- die Formel (Z2)
= [CH-R₂-CH] = (Z2),
in welcher R₂
* einen Cyclus mit der Formel 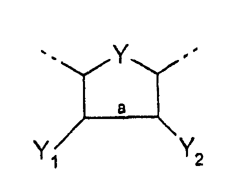 bedeutet, in welcher
• Y -CH₂-, ein Heteroatom, eine -CHR- oder -CHX-Gruppe bedeutet, wobei R und X wie in Anspruch 1 definiert sind,
• Y₁ und Y₂ unabhängig voneinander H oder eine Kette R bzw. Gruppe X, weiter sie weitern oben erwähnt ist, bedeuten oder zusammen mit den Kohlenstoffatomen, die sie tragen, eine Cyclus mit 4 bis 8 Kohlenstoffatomen bilden, wobei dieser Cyclus gegebenenfalls mit einer Kette R oder Gruppe X, wie sie weiter oben erwähnt ist, substituiert ist, und
• a eine Einfach- oder Doppelbindung bedeutet,
* oder einen Cyclus mit der Formel 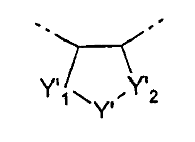 bedeutet, in welcher:
• Y' -CH₂-, ein Heteroatom, eine -CHR- oder -CHX-Gruppe bedeutet, wobei R und X wie weiter oben definiert sind, und
• Y'₁ und Y'₂ unabhängig voneinander -CH₂- oder eine Gruppe -C(O), -COR oder -C-OX bedeuten, wobei R und X wie weiter oben definiert sind, und
- die Formel (Z3) 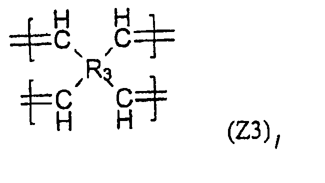 in welcher R₃
* einen Cyclus mit der Formel 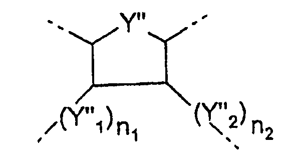 bedeutet, in welcher:
• n₁ und n₂ unabhängig voneinander 0 oder 1 bedeuten,
• Y" -CH₂-, eine -CHR- oder -CHX-Gruppe bedeutet, wobei R und X wie weiter oben definiert sind, und
• Y"₁ und Y"₂ unabhängig voneinander eine Kohlenwasserstoffkette mit 0 bis 10 Kohlenstoffatomen bedeuten,
* einen Cyclus mit der Formel 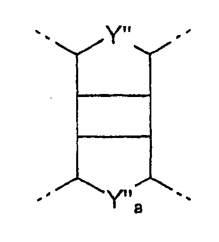 bedeutet, in welcher Y" und Y"a unabhängig voneinander -CH₂-, eine -CHR- oder -CHX-Gruppe bedeuten, wobei R und X wie weiter oben definiert sind, oder
* einen Cyclus mit der Formel 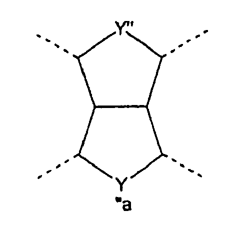 bedeutet, in welcher Y" und Y"a unabhängig voneinander -CH₂-, eine -CHR- oder -CHX-Gruppe bedeuten, wobei R und X wie weiter oben definiert sind, besitzen.

5. Biomaterial nach Anspruch 1 oder 4, **dadurch gekennzeichnet, dass** die polycyclischen Alkene, aus denen die Monomereinheiten hervorgehen:
- Monomere, die einen Cyclobutencyclus enthalten, der ein Polymer ergibt, das Monomereinheiten mit der Formel (Z2a) 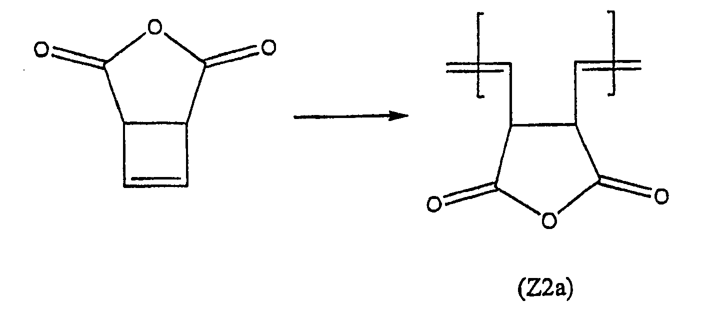 umfasst,
- Monomere, die einen Cyclopentencyclus enthalten, der ein Polymer ergibt, das Monomereinheiten mit der Formel (Z2b) 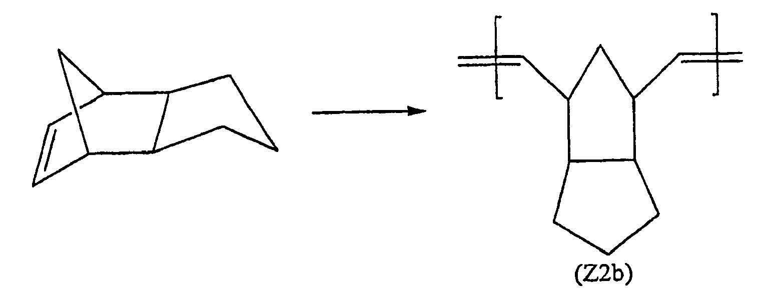 umfasst,
- Norbornen (Bicyclo[2.2.1]hept-2-en), das ein Polymer ergibt, das Monomereinheiten mit der Formel (Z2c) 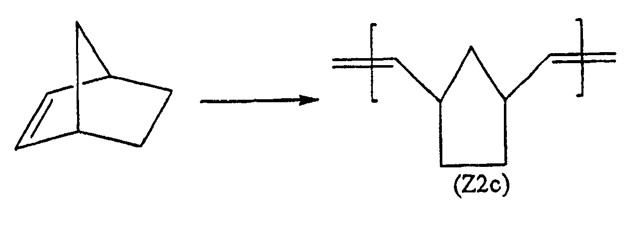 umfasst,
- Norbornadien, das ein Polymer ergibt, das Monomereinheiten mit der Formel (Z2d) 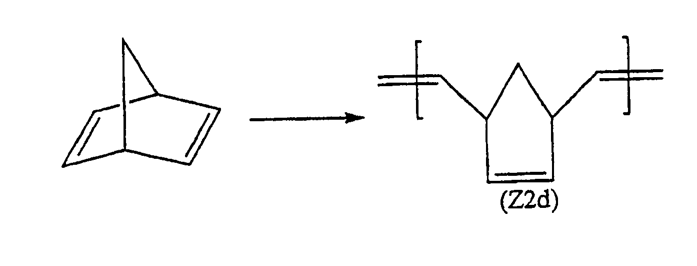 umfasst,
- 7-Oxanorbornen, das ein Polymer ergibt, das Monomereinheiten mit der Formel (Z2e) 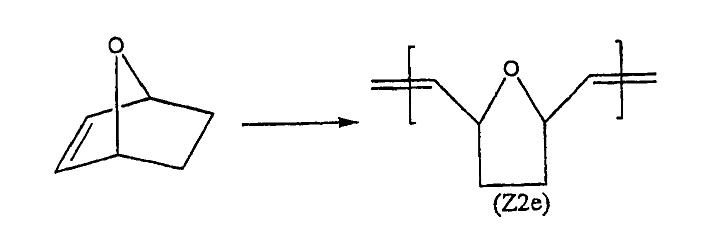 umfasst,
- 7-Oxanorbornadien, das ein Polymer ergibt, das Monomereinheiten mit der Formel (Z2f) 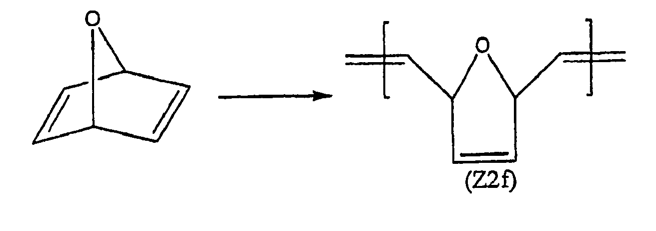 umfasst,
- Norbornadien-Dimer, das ein Polymer ergibt, das Monomereinheiten mit der Formel (Z3a) 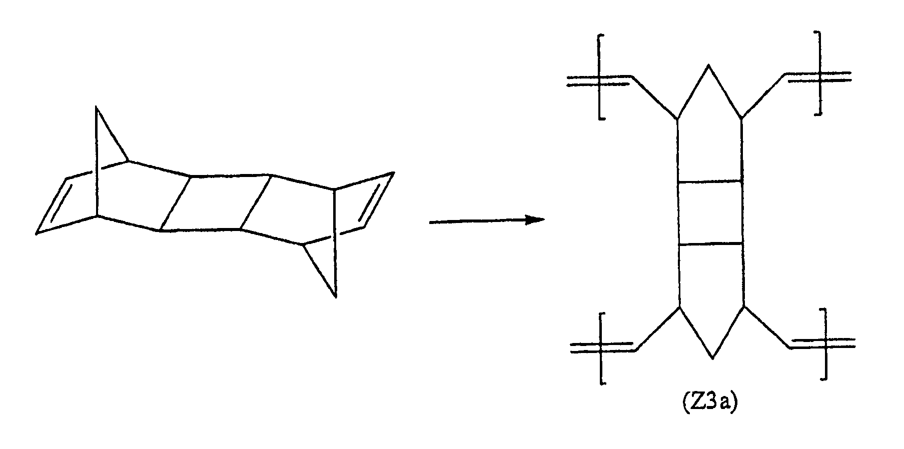 umfasst,
- Dicyclopentadien, das ein Polymer ergibt, das Monomereinheiten mit der Formel (Z3b) 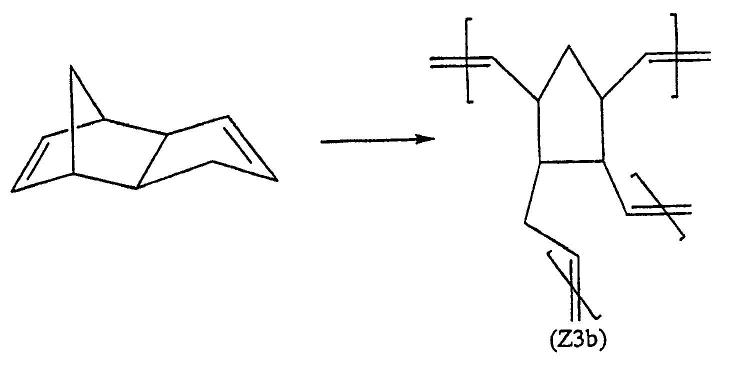 umfasst,
- Tetracyclododecadien, das ein Polymer ergibt, das Monomereinheiten mit der Formel (Z3c) 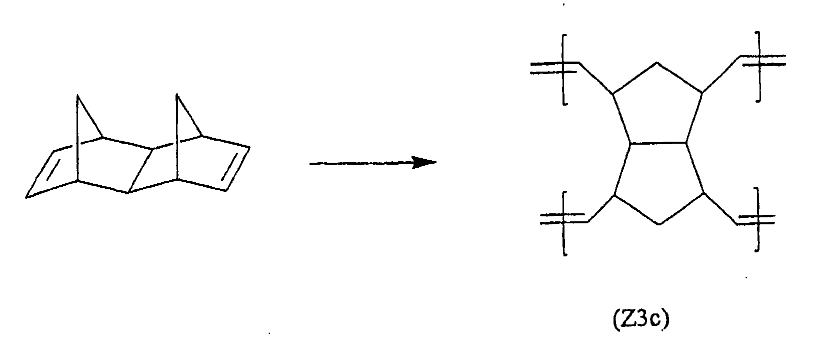 umfasst,
Bicyclo[5.1.0]oct-2-en oder Bicyclo[6.1.0]non-4-en sind.

6. Biomaterial nach einem der Ansprüche 1 bis 5, **dadurch gekennzeichnet, dass** die monocyclischen oder polycyclischen Alkene, aus denen die Monomereinheiten hervorgehen:
- Norbornen (Bicyclo[2.2.1]hept-2-en), das ein Polymer ergibt, das Monomereinheiten mit der Formel (Z2c) umfasst,
- Tetracyclododecadien, das ein Polymer ergibt, das Monomereinheiten mit der Formel (Z3c) umfasst,
- Dicyclopentadien, das ein Polymer ergibt, das Monomereinheiten mit der Formel (Z3b) umfasst,
- Norbornadien-Dimer, das ein Polymer ergibt, das Monomereinheiten mit der Formel (Z3a) umfasst, und
- Cycloocta-1,5-dien, das ein Polymer ergibt, das Monomereinheiten mit der Formel (Z1i) umfasst,
sind.

7. Biomaterial nach einem der Ansprüche 1 bis 6, **dadurch gekennzeichnet, dass** die kugelförmigen Teilchen:
- 0,5 bis 100 % Monomereinheiten, die mit einer wie in Anspruch 1 definierten Kette R substituiert sind, die für diese Monomere identisch ist und eine reaktive Funktion umfasst, die in der Lage ist, sich mit einer reaktiven Funktion, die sich auf dem Trägermaterial befindet, zu verbinden, um die kovalente Bindung dieses Materials mit diesen Teilchen sicherzustellen,
- zwischen 0,5 und 99,5 % Monomereinheiten, die mit einer wie in Anspruch 1 definierten Kette R substituiert sind, die für diese Monomere identisch ist und in welcher die reaktive Funktion eine Bindung mit einem Wirkstoff oder einem biologischen Molekül wie einem Protein eingegangen ist, und/oder
- zwischen 0,5 und 99,5 % Monomereinheiten, die direkt mit einer wie in Anspruch 1 definierten Gruppe X substituiert sind, wobei diese Gruppe X dieser Monomeren sich gegebenenfalls von der Gruppe X der Kette R der vorhergehenden Monomeren unterscheidet, und/oder
- zwischen 1 und 99,5 % unsubstituierte Monomereinheiten, wobei der gesamte Prozentanteil der zuvor genannten verschiedenen Monomeren 100 % beträgt,
umfassen.

8. Biomaterial nach einem der Ansprüche 1 bis 7, **dadurch gekennzeichnet, dass** die die Monomere substituierenden Ketten R die Formel -CH₂-O-(CH₂-CH₂-O)ₙ-CH₂-CH₂-O-X besitzen, in welcher n wie in Anspruch 1 definiert ist und X H, -CH₂-COOH, -CH₂-COCl oder -CH₂-COY bedeutet, wobei Y einen Wirkstoff oder ein biologisches Molekül wie ein Protein bedeutet.

9. Biomaterial nach einem der Ansprüche 1 bis 8, **dadurch gekennzeichnet, dass** die Kette/n R ein Polyethylenoxid mit der Formel A umfasst/umfassen, das mit einer hydrolysierbaren Brücke kovalent verbunden ist, die aus Verknüpfungen mit 1 bis 10 Grundeinheiten ε-Caprolacton oder -OC(O)-, -C(O)OC(O)- und -C(O)-NH-Funktionen ausgewählt ist.

10. Biomaterial nach einem der Ansprüche 1 bis 9, **dadurch gekennzeichnet, dass** die Kette/n R, die ein Polyethylenoxid mit der Formel (A) umfasst/umfassen, das mit einer hydrolysierbaren Brücke kovalent verbunden ist, die aus Verknüpfungen mit 1 bis 10 ε-Caprolacton-Grundeinheiten ausgewählt ist, die Formel -CH₂-(O-CO-(CH₂)₅)ₜ-O-CO-(CH₂)₅-O-CO-(CH₂)₂-CO-O-(CH₂-CH₂-O)ₙ-(CH₂)₂-O-X, in welcher t eine ganze Zahl von 1 bis 10 und X H, -CH₂-COOH, -CH₂-COCl oder -CH₂-COY bedeutet, wobei Y einen Wirkstoff oder ein biologisches Molekül wie ein Protein bedeutet, besitzt/besitzen.

11. Biomaterial nach einem der Ansprüche 1 bis 10, **dadurch gekennzeichnet, dass** das Trägermaterial aus:
- Metallen wie Titan,
- Metalllegierungen, insbesondere gegebenenfalls Legierungen mit Formgedächtnis wie Ni-Ti-Legierungen,
- Polymeren wie Polyethylenterephthalat (PET), Polytetrafluorethylen (PTFE), Polyvinylidenfluorid (PVDF) und Polyetheretherketon (PEEK),
- Copolymeren wie Ethylen-Vinylacetat-Copolymer (EVA), Vinylidenfluorid-Hexafluorpropylen-P-Copolymer (VDF-HFP) und Polymilchsäure-co-polyglykolsäure (PLA-PGA) und
- keramischen Werkstoffen wie Hydroxyapatiten oder Hydroxyapatit- und Tricalciumphosphatverbindungen in verschiedenen Anteilen, insbesondere im Anteil 50/50,
ausgewählt ist.

12. Biomaterial nach einem der Ansprüche 1 bis 11, **dadurch gekennzeichnet, dass** die reaktive Funktion, die sich auf dem Trägermaterial befindet, um die kovalente Bindung dieses Materials mit den Teilchen sicherzustellen, indem sie mit der reaktiven Funktion letzterer reagiert, vom Typ OH, Halogen, NH₂ und C(O)X'₁ ist, in welcher X'₁ ein Wasserstoffatom, ein Halogenatom, eine OR"- oder NHR"-Gruppe bedeutet, in welcher R" ein Wasserstoffatom oder eine gegebenenfalls substituierte Kohlenwasserstoffkette mit 1 bis 10 Kohlenstoffatomen bedeutet, um eine Bindung vom Typ -O-C(O)-, -NH-C(O)-, -C(O)-NH-, -C(O)O- oder -C(OC)₂- mit der reaktiven Funktion dieser Teilchen zu bilden.

13. Biomaterial nach einem der Ansprüche 1 bis 12, **dadurch gekennzeichnet, dass** sich die reaktive Funktion des Trägermaterials an einer gegebenenfalls substituierten Alkylkette mit 1 bis 10 Kohlenstoffatomen befindet, die auf dieses Material gepfropft ist und gegebenenfalls ein oder mehrere Heteroatome, insbesondere O und Si, in der Kette enthält.

14. Biomaterial nach einem der Ansprüche 1 bis 13, **dadurch gekennzeichnet, dass**:
- die reaktive Funktion des Materials eine NH₂-Funktion ist, die sich an einem Aminopropyltriethoxysilanmolekül befindet, das auf das Material gemäß folgender Formeln gepfropft ist: 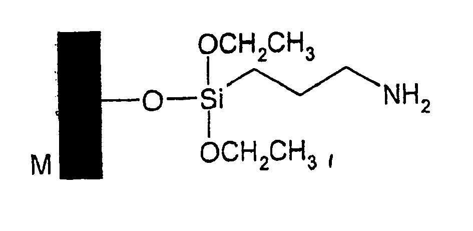 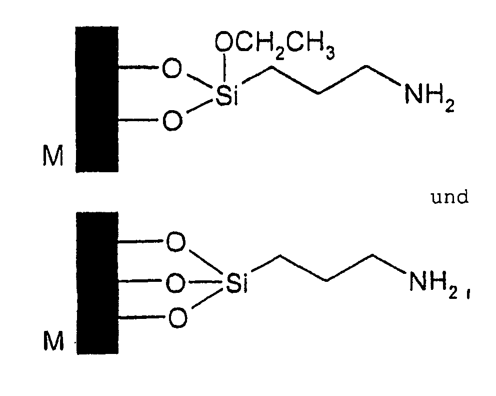 in welchen M ein Metalloxid, ein keramisches Material wie Hydroxyapatit oder ein beliebiges anderes Polymer, die (natürlicherweise oder über eine Präfunktionalisierung) OH-Stellen auf ihrer Oberfläche umfassen, bedeutet, und
- die reaktive Funktion des Materials eine NH₂-Funktion ist, die sich auf einer Oberfläche befindet, die durch Acrylsäure, die mit einem bifunktionellen Abstandshalter wie bNH2PEG (O,O'-Bis(2-aminopropyl)-polyethylenglykol 500) gekoppelt ist, präfunktionalisiert ist.

15. Biomaterial nach einem der Ansprüche 1 bis 14, **dadurch gekennzeichnet, dass** der Wirkstoff aus Molekülen ausgewählt ist, die bei Therapeutika, Kosmetika und Parfümeriewaren oder für Oberflächen-Beschichtungen wie Anstrichstoffe und fäulnisverhindernde Mittel verwendet werden.

16. Biomaterial nach einem der Ansprüche 1 bis 15, **dadurch gekennzeichnet, dass** der Wirkstoff aus Molekülen, die bei Therapeutika verwendet werden, insbesondere aus denjenigen der folgenden Therapeutikaklassen: Antibiotika, entzündungshemmenden Mitteln, Antimitotika, Hormonen und Wachstumsfaktoren ausgewählt ist.

17. Verwendung von Biomaterialien nach einem der Ansprüche 1 bis 16 zur Herstellung von implantierbaren medizinischen Vorrichtungen, insbesondere in Form von Implantaten, Prothesen und Stents, oder von Zementen, insbesondere für die endovaskuläre, Gefäß- oder Knochenchirurgie.

18. Implantate, Prothesen, Gefäßstents oder Zemente, die Biomaterialien nach einem der Ansprüche 1 bis 16 umfassen.
